# EUROPEAN PATENT SPECIFICATION

(11) **EP 3 735 219 B1**
(45) Date of publication and mention of the grant of the patent: **16.10.2024**
(21) Application number: 19703787.2
(22) Date of filing: 02.01.2019
(51) Int. Cl.: A61K 9/00, A61K 9/10, A61K 9/16, A61K 9/50, A61K 31/606, A61P 1/00

(54) **ORAL LIQUID PHARMACEUTICAL COMPOSITIONS OF AMINOSALICYLATES**
ORALE FLÜSSIGE PHARMAZEUTISCHE ZUSAMMENSETZUNGEN VON AMINOSALICYLATEN
COMPOSITIONS PHARMACEUTIQUES LIQUIDES ORALES D'AMINOSALICYLATES

(30) Priority: 03.01.2018 US 201862613198 P
(43) Date of publication of application: 11.11.2020
(73) Proprietor: Ferring B.V., 2132 JX Hoofddorp (NL)
(72) Inventor: LIANG, Alfred Chi-Yeh, 2132 JX Hoofddorp (NL); EHRNSPERGER, Eric, 2132 JX Hoofddorp (NL); SHEN, Xiaohong, 2132 JX Hoofddorp (NL)
(74) Representative: Hoffmann Eitle
(86) International application number: PCT/US2019/012032
(87) International publication number: WO 2019/136053

(56) References cited:
- EP-A1- 2 229 937
- WO-A1-2017/012935
- WO-A1-95/05806
- WO-A2-2005/021009

## Description

### FIELD

Described herein are oral liquid pharmaceutical compositions for the oral administration of aminosalicylates, such as mesalazine, as well as methods of making such oral liquid pharmaceutical compositions, and therapeutic methods using them.

### BACKGROUND

Aminosalicylates are anti-inflammatory drugs used to treat conditions such as inflammatory bowel disease, such as ulcerative colitis and mild-to-moderate Crohn's disease. Examples of aminosalicylates include 4-aminosalicylic acid, balsalazide, olsalazine, sulfasalazine, and mesalazine. The activity of aminosalicylates against these conditions is primarily local, and so aminosalicylates typically are administered by a dosage form and route of administration that will deliver the aminosalicylate to a desired site of activity, such as the colon or small bowel, for example. Thus, aminosalicylates are available in various dosage forms, including oral and rectal formulations, including formulations having different release mechanisms, such as time-dependent/pH-independent release mechanisms (such as Pentasa^{®}) and pH-dependent release mechanisms (such as Asacol^{®} or Delzicol^{®}) designed to release the aminosalicylates at an intended site of action. Other dosage forms of aminosalicylates include Lialda^{®}, Dipentum^{®}, Colazal^{®}, and Giazo^{®}. Currently available oral dosage forms of aminosalicylates include solid dosage forms, such as tablets, capsules and sachets. Thus, there remains a need for liquid pharmaceutical compositions for the oral administration of aminosalicylates.

### SUMMARY

Provided herein are oral liquid pharmaceutical compositions comprising a plurality of extended release aminosalicylate microparticles provided with an enteric delayed release coating, suspended in an aqueous liquid carrier formulated for oral administration, as defined in the claims.

In any embodiments, the aminosalicylate may comprise one or more of mesalazine, 4-aminosalicylic acid, balsalazide, olsalazine, sulfasalazine, or a pharmaceutically acceptable salt of any one more thereof. In some embodiments, the aminosalicylate comprises mesalazine or a pharmaceutically acceptable salt thereof.

In any embodiments, the Dx90 particle size of the extended release aminosalicylate microparticles provided with a delayed release coating may be from about 100 µm to about 250 µm, or ≤ 250 µm, ≤ 200 µm, ≤ 150 µm, or ≤ 100 µm.

In any embodiments, the extended release aminosalicylate microparticles may be prepared by a process comprising dispersing an aminosalicylate in a solution comprising an extended release polymer to obtain a dispersed phase, emulsifying the dispersed phase in an immiscible solvent, and evaporating the solvent from the dispersed phase, to obtain a plurality of the extended release aminosalicylate microparticles.

In any embodiments, the delayed release coating is an enteric coating comprising one or more delayed release polymers selected from copolymers of methacrylic acid and methyl methacrylate (such as Eudragit^{®} L 100 and/or Eudragit^{®} S 100).

In some embodiments, the delayed release coating comprises two or more delayed release polymers selected from copolymers of methacrylic acid and methyl methacrylate.

In some embodiments, the delayed release coating comprises a first delayed release polymer and a second delayed release polymer at a ratio of from 10:90 to 90: 10. In some embodiments, the delayed release coating comprises poly(methacrylic acid-co-methyl methacrylate) 1: 1 and poly(methacrylic acid-co-methyl methacrylate) 1:2. In some embodiments, the delayed release coating comprises poly(methacrylic acid-co-methyl methacrylate) 1: 1 and poly(methacrylic acid-co-methyl methacrylate) 1:2 at a ratio of 1:1.

In some embodiments, the delayed release coating is provided as a bilayer comprised of a first layer comprising one or more delayed release polymers and a second layer comprising one or more delayed release polymers.

In some embodiments, the delayed release coating is provided on the extended release aminosalicylate microparticles by a process comprising dispersing the extended release aminosalicylate microparticles in a solution comprising delayed release polymer to obtain a dispersed phase, emulsifying the dispersed phase in an immiscible solvent, and evaporating the solvent from the dispersed phase, to obtain a plurality of extended release aminosalicylate microparticles with an outer delayed release coating.

In some embodiments, the delayed release coating is provided on the extended release aminosalicylate microparticles by a process comprising fluid bed coating. In specific embodiments, the delayed release coating is provided on the extended release aminosalicylate microparticle by a process comprising dissolving one or more delayed release polymers in a suitable solvent to obtain a delayed release polymer solution, and spray-coating the extended release aminosalicylate microparticles with the delayed release polymer solution in a fluid bed coater to obtain a plurality of extended release aminosalicylate microparticles with a delayed release coating.

In one embodiment, the oral liquid pharmaceutical composition comprises about 4-8% w/w aminosalicylate, about 4-8% w/w poly(methacrylic acid-co-methyl methacrylate) 1:2, about 5-12% w/w cellulose acetate, about 0.8-1.2% w/w microcrystalline cellulose, about 0.05-0.1% w/w xanthan gum, and water.

In another embodiment, the oral liquid pharmaceutical composition comprises about 2-6% w/w aminosalicylate, about 6-10% w/w poly(methacrylic acid-co-methyl methacrylate) 1:2, about 5-12% w/w cellulose acetate butyrate, about 0.8-1.2% w/w microcrystalline cellulose, about 0.05-0.1% w/w xanthan gum, and water.

In another embodiment, the oral liquid pharmaceutical composition comprises about 4-5% w/w aminosalicylate, about 3-8% w/w poly(methacrylic acid-co-methyl methacrylate) 1:1, about 3-8% w/w poly(methacrylic acid-co-methyl methacrylate) 1:2, about 3-8% w/w cellulose acetate butyrate, about 0.5-1.5% w/w microcrystalline cellulose, about 0.05-0.1% w/w xanthan gum, and water.

In another embodiment, the oral liquid pharmaceutical composition comprises about 4-5% w/w aminosalicylate, about 4-5% w/w poly(methacrylic acid-co-methyl methacrylate) 1:1, about 1-5% w/w poly(methacrylic acid-co-methyl methacrylate) 1:2, about 3-9% w/w cellulose acetate butyrate, about 0.5-1.5% w/w microcrystalline cellulose, 0.05-0.1% w/w xanthan gum, and water.

In any embodiments, the oral liquid pharmaceutical composition contains 2 g aminosalicylate in a volume selected from about 30 to about 60 mL, or 60 mL or less, 50 mL or less, 45 mL or less, 40 mL or less, 35 mL or less, or 30 mL or less.

In accordance with the present invention, the oral liquid pharmaceutical composition exhibits an *in vitro* dissolution profile such that, when subject to *in vitro* dissolution testing according to USP II Paddle at 37 °C and 100 rpm in 750 mL dissolution medium of pH 1.2 buffer for 2 hours, followed by pH 7 buffer for 12 hours, the composition exhibits substantially no release of aminosalicylate at pH 1.2, and releases substantially all aminosalicylate within about 3 to about 8 hours at pH 7.

In accordance with any embodiments described herein, the extended release aminosalicylate microparticles provided with a delayed release coating may exhibit an *in vitro* dissolution profile such that, when subject to *in vitro* dissolution testing according to USP II Paddle at 37 °C and 100 rpm in 750 mL dissolution medium of pH 1.2 buffer for 2 hours, followed by pH 7 buffer for 12 hours, the composition exhibits substantially no release of aminosalicylate at pH 1.2, and releases substantially all aminosalicylate within about 3 to about 8 hours at pH 7.

In accordance with any embodiments described herein, the oral liquid pharmaceutical composition may be stable for at least 1 year or at least 2 years when stored at 25°C and 60% relative humidity. In accordance with any embodiments described herein, after storage for 2 years at 25 °C and 60% relative humidity, the oral liquid pharmaceutical composition may exhibit an *in vitro* dissolution profile such that, when subject to in vitro dissolution testing according to USP II Paddle at 37 °C and 100 rpm in 750 mL dissolution medium of pH 1.2 buffer for 2 hours, followed by pH 7 buffer for 12 hours, the oral liquid pharmaceutical composition exhibits substantially no release of aminosalicylate at pH 1.2, and releases substantially all aminosalicylate within about 3 to about 8 hours at pH 7. In accordance with any embodiments described herein, after storage for 2 years at 25 °C and 60% relative humidity, the amount of aminosalicylate degradants in the composition may be less than 3% by weight of the original amount of aminosalicylate in the composition. In accordance with any embodiments described herein, after storage for 2 years at 25 °C and 60% relative humidity, the amount of aminosalicylate present in the microparticles of the oral liquid pharmaceutical composition differs by no more than 3% by weight from the original amount.

Also provided are processes for making an oral liquid pharmaceutical aminosalicylate composition as described herein, comprising (a) preparing a plurality of extended release aminosalicylate microparticles; (b) providing an outer delayed release coating on the extended release aminosalicylate microparticles to obtain a plurality of delayed/extended release aminosalicylate microparticles; and (c) suspending the plurality of delayed/extended release aminosalicylate microparticles in an aqueous liquid carrier, as defined in the claims.

In some embodiments, step (a) comprises an emulsion solvent evaporation process, such as a process comprising dispersing aminosalicylate in a solution comprising an extended release polymer to obtain a dispersed phase, emulsifying the dispersed phase in an immiscible solvent, and evaporating the solvent from the dispersed phase, to obtain the plurality of extended release aminosalicylate microparticles.

In some embodiments, step (b) comprises an emulsion solvent evaporation process, such as a process comprising dispersing the extended release aminosalicylate microparticles in a solution comprising delayed release polymer to obtain a dispersed phase, emulsifying the dispersed phase in an immiscible solvent, and evaporating the solvent from the dispersed phase, to obtain the delayed/extended release aminosalicylate microparticles. In some embodiments, step (b) comprises a fluid bed coating process, optionally followed by an oven curing process. In some embodiments, the method comprises curing the delayed/extended release aminosalicylate microparticles after step (b) before step (c).

In some embodiments, the process further comprises prior to step (c), screening the delayed/extended release aminosalicylate microparticles to select delayed/extended release aminosalicylate microparticles having a Dx90 particle size of from about 100 µm to about 250 µm, or ≤ 250 µm, ≤ 200 µm, ≤ 150 µm, or ≤ 100 µm, and performing step (c) on the selected delayed/extended release aminosalicylate microparticles.

Also provided are oral liquid pharmaceutical compositions as described herein for use in treating inflammatory bowel disease, such as ulcerative colitis and mild-to-moderate Crohn's disease.

### BRIEF DESCRIPTION OF THE DRAWINGS

FIG. 1 is a flow chart for the manufacture of extended release aminosalicylate microparticles using oil-in-oil solvent evaporation technology as described herein.
FIG. 2 is a flow chart for the manufacture of extended release aminosalicylate microparticles using oil-in-water solvent evaporation technology as described herein.
FIG. 3 is a flow chart for the manufacture of delayed/extended release aminosalicylate microparticles using oil-in-oil solvent evaporation technology as described herein.
FIG. 4 is a graphical depiction of an *in vitro* dissolution profile of delayed/extended release aminosalicylate microparticles obtained using oil-in-oil solvent evaporation technology as described herein.
FIG. 5 is a graphical depiction of an *in vitro* dissolution profile of delayed/extended release aminosalicylate microparticles obtained using oil-in-water solvent evaporation technology followed by fluid bed coating as described herein.
FIG. 6 is a graphical depiction of an *in vitro* dissolution profile of an oral liquid pharmaceutical composition as described herein.
FIG. 7 is a graphical depiction of an *in vitro* dissolution profile of an oral liquid pharmaceutical composition as described herein after storage.
FIG. 8 is a graphical depiction of an *in vitro* dissolution profile of extended release aminosalicylate microparticles obtained using oil-in-water solvent evaporation technology as described herein.
FIG. 9 is a graphical depiction *of in vitro* dissolution profiles of liquid pharmaceutical compositions comprising delayed/extended release aminosalicylate microparticles having a delayed-release coating containing a 1:1 mixture of Eudragit^{®} L100 and Eudragit^{®} S100, where the delayed/extended release microparticles were either cured or not cured prior to formulation in a liquid suspension.
FIG. 10 is a graphical depiction of *in vitro* dissolution profiles of oral liquid pharmaceutical compositions comprising delayed/extended release aminosalicylate microparticles with a delayed-release bilayer coating comprised of an Eudragit^{®} L100 layer (80% weight gain) and an Eudragit^{®} S100 layer (20% weight gain), where the delayed/extended release microparticles were either cured or not cured prior to formulation in a liquid suspension.
FIG. 11 is a graphical depiction of an *in vitro* dissolution profile of extended release aminosalicylate microparticles obtained using oil-in-water solvent evaporation technology as described herein.
FIG. 12 is a graphical depiction of *in vitro* dissolution profiles of oral liquid pharmaceutical compositions comprising delayed/extended release aminosalicylate microparticles with a delayed-release coating containing a 1:1 mixture of Eudragit^{®} L100 and Eudragit^{®} S100, where the delayed/extended release microparticles were either cured or not cured prior to formulation in a liquid suspension.
FIG. 13 is a graphical depiction of an *in vitro* dissolution profile of extended release aminosalicylate microparticles obtained using oil-in-water solvent evaporation technology as described herein.
FIG. 14 is a graphical depiction of *in vitro* dissolution profiles of oral liquid pharmaceutical compositions comprising delayed/extended release aminosalicylate microparticles with a delayed release coating containing a 1:1 mixture of Eudragit^{®} L100 and Eudragit^{®} S100, where the delayed/extended release microparticles were either cured or not cured prior to formulation in a liquid suspension.
FIG. 15 is a graphical depiction of *in vitro* dissolution profiles of an oral liquid pharmaceutical composition comprising delayed/extended release aminosalicylate microparticles with a delayed-release coating containing a 1:1 mixture of Eudragit^{®} L100 and Eudragit^{®} S100 before and after storage at 40 °C and 75% RH, where the delayed/extended release microparticles were cured prior to formulation in a liquid suspension.
FIG. 16 is a graphical depiction *of in vitro* dissolution profiles of an oral liquid pharmaceutical composition comprising delayed/extended release aminosalicylate microparticles with a delayed-release coating containing a 1:1 mixture of Eudragit^{®} L100 and Eudragit^{®} S100 before and after storage at 30 °C and 65% RH, where the delayed/extended release microparticles were cured prior to formulation in a liquid suspension.
FIG. 17 is a graphical depiction *of in vitro* dissolution profiles of an oral liquid pharmaceutical composition comprising delayed/extended release aminosalicylate microparticles with a delayed-release coating containing a 1:1 mixture of Eudragit^{®} L100 and Eudragit^{®} S100 before and after storage at 25 °C and 60% RH, where the delayed/extended release microparticle were cured prior to formulation in a liquid suspension.

### DETAILED DESCRIPTION

Described herein are oral liquid pharmaceutical compositions for the oral administration of aminosalicylates, methods of making such oral liquid pharmaceutical compositions, and therapeutic methods for using them. In accordance with some embodiments, the oral liquid pharmaceutical compositions deliver an aminosalicylate to the lower gastrointestinal tract, such as the distal ileum and/or the colon, over a period of time sufficient to achieve therapeutic effect. In accordance with specific embodiments, there are provided oral liquid pharmaceutical compositions comprising a plurality of extended release aminosalicylate microparticles provided with a delayed release coating, suspended in an aqueous liquid carrier formulated for oral administration, as defined in the claims.

### Definitions

Technical and scientific terms used herein have the meanings commonly understood by one of ordinary skill in the art of pharmaceutical formulations to which the present disclosure pertains, unless otherwise defined. Reference is made herein to various methodologies known to those of ordinary skill in the art. Suitable materials and/or methods known to those of ordinary skill in the art can be utilized in carrying out the present disclosure. However, specific materials and methods are described. Materials, reagents and the like to which reference is made in the following description and examples are obtainable from commercial sources, unless otherwise noted.

As used herein, the singular forms "a," "an," and "the" designate both the singular and the plural, unless expressly stated to designate the singular only.

As used herein, the term "about" means that the number or range is not limited to the exact number or range set forth but encompass values around the recited number or range as will be understood by persons of ordinary skill in the art depending on the context in which the number or range is used. Unless otherwise apparent from the context or convention in the art, "about" means up to plus or minus 10% of the particular term.

As used herein, "subject" denotes any mammal, including humans. For example, a subject may be suffering from or at risk of developing a condition that can be diagnosed, treated or prevented with mesalazine, or may be taking mesalazine for other purposes.

The terms "administer," "administration," or "administering" as used herein refer to (1) providing, giving, dosing and/or prescribing, such as by either a health professional or his or her authorized agent or under his direction, and (2) putting into, taking or consuming, such as by a health professional or the subject.

The terms "treat", "treating" or "treatment", as used herein, include alleviating, abating or ameliorating a disease or condition or one or more symptoms thereof, whether or not the disease or condition is considered to be "cured" or "healed" and whether or not all symptoms are resolved. The terms also include reducing or preventing progression of a disease or condition or one or more symptoms thereof, impeding or preventing an underlying mechanism of a disease or condition or one or more symptoms thereof, and achieving any therapeutic and/or prophylactic benefit.

As used herein, the phrase "therapeutically effective amount" refers to a dose that provides the specific pharmacological effect for which the drug is administered in a subject in need of such treatment. It is emphasized that a therapeutically effective amount will not always be effective in treating the conditions described herein, even though such dose is deemed to be a therapeutically effective amount by those of skill in the art. For convenience only, exemplary doses and therapeutically effective amounts are provided below with reference to adult human subjects. Those skilled in the art can adjust such amounts in accordance with standard practices as needed to treat a specific subject and/or condition/disease.

As noted above, in accordance with specific embodiments, there are provided oral liquid pharmaceutical compositions comprising a plurality of extended release aminosalicylate microparticles provided with a delayed release coating, suspended in an aqueous liquid carrier formulated for oral administration. Specific aspects and specific embodiment are discussed in more detail below.

### Aminosalicylates

As noted above, described herein are compositions that include an aminosalicylate (or a pharmaceutically acceptable salt or ester thereof). Aminosalicylates include but are not limited to 4-aminosalicylic acid, balsalazide, olsalazine, sulfasalazine, and/or mesalazine and pharmaceutically acceptable salts and esters of each thereof. Thus, as used herein, the term "aminosalicylate" includes but is not limited to 4-aminosalicylic acid, balsalazide, olsalazine, sulfasalazine, and/or mesalazine, and pharmaceutically acceptable salts and esters of each thereof.

Mesalazine also is known as 5-amino-2-hydroxybenzoic acid, 5-ASA, and mesalamine. Mesalazine and has the molecular formula C₇H₇NO₃ and a molecular weight of 153.14. It is registered under CAS Registry Number 89-57-6 and Einecs 201-919-1.

4-aminosalicylic acid is also known as para-aminosalicylic acid, p-aminosalicylic acid, PAS, and 4-ASA, has the chemical name 4-amino-2-hydroxybenzoic acid, the molecular formula C₇H₇NO₃ and a molecular weight of 153.14. It is registered under CAS Registry Number 65-49-6 and Einecs 200-615-5.

Balsalazide has the chemical name (E)-5-([4-(2-carboxyethylcarbamoyl)phenyl]diazenyl)-2-hydroxybenzoic acid), has the molecular formula C₁₇H₁₅N₃O₆ and a molecular weight of 357.32. It is registered under CAS Registry Number 80573-04-2 and Einecs 617-116-8.

Olsalazine has the chemical names 5-[(2Z)-2-(3-carboxy-4-oxocyclohexa-2,5-dien-1-ylidene)hydrazino]-2-hydroxybenzoic acid and 3,3' -azobis (6-hydroxybenzoate)salicylic acid and has the molecular formula C₁₄H₁₀N₂O₆ and a molecular weight of 302.24. It is registered under CAS Registry Number 15722-48-2 and Einecs 605-089-5.

Sulfasalazine has the chemical name 2-hydroxy-5-[(*E*)-2-{4-[(pyridin-2-yl)sulfamoyl]phenyl}diazen-1-yl]benzoic acid) and has the molecular formula C₁₈H₁₄N₄O₅S and a molecular weight of 398.39. It is registered under CAS Registry Number 599-79-1 and Einecs 209-974-3.

As used herein, the common names (e.g., 4-ASA, balsalazide, olsalazine, sulfasalazine, and/or mesalazine) include pharmaceutically acceptable salts and esters, while use of the chemical names (e.g., 5-amino-2-hydroxybenzoic acid) refers to that compound *per se*, as distinguished from pharmaceutically acceptable salts and esters thereof.

In specific embodiments, the aminosalicylate comprises 4-aminosalicylic acid, balsalazide, olsalazine, sulfasalazine, or mesalazine. In specific embodiments, the aminosalicylate comprises mesalazine. In specific embodiments, the aminosalicylate comprises 4-aminosalicylic acid. In specific embodiments, the aminosalicylate comprises balsalazide. In specific embodiments, the aminosalicylate comprises olsalazine. In specific embodiments, the aminosalicylate comprises sulfasalazine.

Exemplary pharmaceutically acceptable salts include acid addition salts, such as hydrochloride salts. Any pharmaceutically acceptable salt can be used, such as sodium and calcium salts. Other non-limiting exemplary salts include salts formed with a carboxylic acid group, alkali metal salts, and alkaline earth metal salts. Non-limiting examples of pharmaceutically acceptable esters include straight chain or branched C₁-C₁₈ alkyl esters, including methyl, ethyl, propyl, isopropyl, butyl, isobutyl, amyl, hexyl, heptyl, octyl, nonyl, decyl, lauryl, myristyl, cetyl, and stearyl, and the like; straight chain or branched C₂-C₁₈ alkenyl esters, including vinyl, allyl, undecenyl, oleyl, linolenyl, and the like; C₃-C₈ cycloalkyl esters, including cyclopropyl, cyclobutyl, cyclopentyl, cyclohexyl, cycloheptyl and cyclooctyl, and the like; aryl esters, including phenyl, toluoyl, xylyl, naphthyl, and the like; alicyclic esters, including menthyl and the like; or aralkyl esters, including benzyl, phenethyl, and the like.

The compositions described herein may include a therapeutically effective amount of an aminosalicylate. The therapeutically effective amount may depend on the specific aminosalicylate being used, the subject being treated, the condition being treated, the desired effect, and the intended duration of therapeutic effect of the compositions and products. In some embodiments, the oral liquid pharmaceutical compositions described herein are formulated to contains 2 g aminosalicylate in a volume selected from about 30 to about 60 mL, or 60 mL or less, 50 mL or less, 45 mL or less, 40 mL or less, 35 mL or less, or 30 mL or less.

A therapeutically effective amount of orally administered mesalazine for the treatment of inflammatory bowel disease, is generally about 2 to about 6 g/day for an adult human subject, optionally administered in divided two to four doses (e.g., about 4 g once a day, about 2 g two times a day, about 1 g four times a day, *etc*.). For example, therapeutic doses of mesalazine for patients with active ulcerative colitis of any disease extent beyond proctitis is typically from about 2 to about 5 g a day, including from about 2.4 to about 4.8 g/day, with or without concomitant rectal therapy. In some embodiments, the oral liquid pharmaceutical compositions described herein are formulated to contains 2 g mesalazine in a volume selected from about 30 to about 60 mL, or 60 mL or less, 50 mL or less, 45 mL or less, 40 mL or less, 35 mL or less, or 30 mL or less. Doses may be adjusted as appropriate for pediatric subjects.

### Extended Release Aminosalicylate Microparticles

The extended release aminosalicylate microparticles comprise an aminosalicylate formulated in an extended release matrix comprising an extended release polymer. Extended release polymers suitable for this purpose are known in the art and include hydrophobic polymers with solubility profiles that are substantially flat over physiological pH (e.g., over pH 1-7.5). Extended release polymers used for this purpose in the present invention are one or more selected from cellulose acetate polymers and cellulose acetate butyrate polymers.

The uncoated extended release aminosalicylate microparticles may comprise aminosalicylate in an amount from about 30 to about 60% by weight, or about 60% by weight or less, about 55% by weight or less, about 50% by weight or less, about 45% by weight or less, about 40% by weight or less, about 35% by weight or less, about 30% by weight or less, based on the uncoated weight of the extended release microparticles.

In specific embodiments, the aminosalicylate of the extended release microparticles is mesalazine and is present in an amount from about 30 to about 60% by weight, based on the uncoated weight of the extended release microparticles, including from 35% to 55% by weight, including 50% by weight.

The extended release aminosalicylate microparticles can be made by a process described below. In some embodiments, the extended release aminosalicylate microparticles are sized such that, after being provided with a delayed release coating as discussed below, they have a Dx90 particle size of ≤ 200 µm, such as from about 100 µm to about 200 µm, including ≤ 200 µm, ≤ 150 µm or ≤ 100 µm. For example, in some embodiments, the extended release aminosalicylate microparticles have a Dx90 particle size of ≤ 150 µm, such as from about 50 µm to about 150 µm, including ≤ 150 µm, and ≤ 100 µm.

### Delayed/Extended Release Aminosalicylate Microparticles

The extended release aminosalicylate microparticles described herein are provided with a delayed release coating. Such coated extended release aminosalicylate microparticles also are referred to herein as delayed/extended release aminosalicylate microparticles.

In the present invention, the delayed release coating is an enteric coating, e.g., a coating whose solubility varies with pH and is designed to dissolve at a pH associated with the target site of activity, such as the lower gastrointestinal tract, such as a pH of about 5.5 or greater, including a pH of about 7 or greater.

Suitable delayed-release polymers for enteric coatings are known in the art. Commercially available enteric polymers include those sold by Evonik Industries under the Eudragit^{®} trademark, including Eudragit^{®} L (poly(methacrylic acid, methyl methacrylate) 1: 1), and Eudragit^{®} S (poly(methacrylic acid, methyl methacrylate) 1:2 ). For example, Eudragit^{®} L 30 D-55 (poly(methacrylic acid-co-ethyl acrylate) 1: 1) and Eudragit^{®} L-100-55 (poly(methacrylic acid-co-ethyl acrylate) 1:1) are reported to dissolve above pH 5.5, Eudragit^{®} L 100 (poly(methacrylic acid-co-methyl methacrylate) 1: 1) and Eudragit^{®} L 12.5 (poly(methacrylic acid-co-methyl methacrylate) 1:1) are reported to dissolve above pH 6.0, and Eudragit^{®} S 100 (poly(methacrylic acid-co-methyl methacrylate) 1:2), Eudragit^{®} S 12.5 (poly(methacrylic acid-co-methyl methacrylate) 1:2) and Eudragit^{®} FS 30D (poly(methyl acrylate-co-methyl methacrylate-co-methacrylic acid) 7:3:1) are reported to dissolve above pH 7.0. Another commercially available enteric polymer is sold by ShinEtsu as AQOAT^{®} (hypromellose acetate succinate).

pH-dependent enteric coatings may include a single enteric polymer or a mixture of enteric polymers. The relative amounts of the enteric polymers in the delayed release coating and the thickness at which the coating is provided on the microparticles can be independently selected to achieve release at the intended pH, e.g., at the intended site of the gastrointestinal tract. For example, the polymer(s) can be selected and combined in relative amounts and provided at a thickness to achieve dissolution at the target pH, e.g., at a pH of about 5.5 or a pH of about 7 or greater.

The delayed release coating may include a single delayed release polymer or a mixture of delayed release polymers, including any one or more of the enteric polymers discussed above. The identity and relative amounts of the delayed release polymers in the delayed release coating and the thickness at which the coating is provided on the microparticles can be independently selected to achieve release at the intended pH, or to achieve other desired properties, such as stability.

In some embodiments, the delayed release coating is applied at a weight gain of from about 75% to about 125%, based on the weight of the uncoated microparticles, including a weight gain of about 100%.

In the present invention, the delayed release coating comprises one or more delayed release polymers selected from copolymers of methacrylic acid and methyl methacrylate (such as Eudragit^{®} L 100 and/or Eudragit^{®} S 100).

In specific embodiments, the delayed release coating comprises two or more delayed release polymers, such as two or more selected from copolymers of methacrylic acid and methyl methacrylate (such as Eudragit^{®} L 100 and Eudragit^{®} S 100). As noted above, the relative amounts of each are selected to achieve specific properties.

In some embodiments, the delayed release coating is provided as a single coating layer, which may comprise one or more delayed release polymers as discussed above. In some embodiments, the delayed release coating is provided as multiple coating layers, such as two, or three, or more, coating layers, which each may comprise one or more delayed release polymers as discussed above, and which each may be applied at a coating thickness (or % weight gain) selected to achieve desired properties.

In some embodiments, the delayed release coating is a bilayer coating where a first layer comprises a first delayed release polymer(s) and a second layer comprises second delayed release polymer(s). In some embodiments, the delayed release coating is a bilayer coating where a first layer comprises a first delayed release polymer and a second layer comprises second delayed release polymer, both selected from copolymers of methacrylic acid and methyl methacrylate (such as Eudragit^{®} L 100 and Eudragit^{®} S 100). In some embodiments, the first delayed release polymer comprises or is poly(methacrylic acid-co-methyl methacrylate) 1: 1 (such as Eudragit^{®} L 100) and the second polymer comprises or is poly(methacrylic acid-co-methyl methacrylate) 1:2 (such as Eudragit^{®} S 100). As noted above, the relative amounts of each can be selected to achieve specific properties. For example, the first and second layers may be applied in relative amounts ranging from about 1:99 to about 99:1, from about 5:95 to about 95:5, from about 10:90 to about 90:10, from about 20:80 to about 80:20; from about 25:75 to about 75:25, from about 33.3:66.7 to about 66.7:33.3, from about 40:60 to about 60:40, or about 1:1.

Any coating described herein may also include an anti-tacking agent and/or a plasticizer. Typical anti-tacking agents used for this purpose include one or more of talc, colloidal silicon dioxide, magnesium aluminum silicate, and calcium stearate. In specific embodiments, a coating includes talc. Typical plasticizers used for this purpose include water-insoluble plasticizers such as one or more of acetyl tributyl citrate, acetyl triethyl citrate, castor oil, diacetylated monoglycerides, dibutyl sebacate, diethyl phthalate, glyceryl monostearate, glyceryl palmitostearate, and sodium stearyl fumarate. In some embodiments, a coating includes a plasticizer, such as dibutyl sebacate or acetyl tributyl citrate.

In specific embodiments, the extended release aminosalicylate microparticles provided with a delayed release coating (i.e., the delayed/extended release aminosalicylate microparticles) have a Dx90 particle size of ≤ 250 µm, such as from about 100 µm to about 250 µm, including ≤ 250 µm, ≤ 200 µm, ≤ 150 µm, or ≤ 100 µm.

In some embodiments, the delayed/extended release aminosalicylate microparticles exhibit an *in vitro* dissolution profile such that, when subject to *in vitro* dissolution testing according to USP II Paddle at 37 °C and 100 rpm in 750 mL dissolution medium of pH 1.2 buffer for 2 hours, followed by pH 7 buffer for 12 hours, the delayed/extended release aminosalicylate microparticles exhibit substantially no release of aminosalicylate at pH 1.2, and release substantially all aminosalicylate within about 3 to about 8 hours, or within about 3 to about 7 hours, at pH 7. As used herein release of "substantially all" aminosalicylate refers to at least 75%, at least 80%, at least 85%, at least 90%, at least 95%, or 100% release, by weight.

### Liquid Carrier

As noted above, in the oral liquid pharmaceutical compositions as described herein, the microparticles are suspended in an aqueous liquid carrier. The liquid carrier described herein may be used to formulate other particulates, including microparticles comprising other active agents.

The aqueous liquid carriers described herein typically include a thixotropic agent and a thickening agent. Suitable thixotropic agents are known in the art, and include microcrystalline cellulose/sodium carboxymethylcellulose products, such as those sold under the Avicel^{®} brand by FMC BioPolymer, such as Avicel^{®} RC 591. Suitable thickening agents are known in the art and include xanthan gum, sodium alginate, methylcellulose, hydroxyethylcellulose, hydroxypropylcellulose, hydroxypropylmethylcellulose, carboxymethylcellulose, tragacanth, bentonite, carrageenan, guar gum, colloidal silicon dioxide, and carbopol polymers.

The identity and amounts of the thixotropic agent and thickening agent can be independently selected and adjusted to achieve the desired viscosity, fluidity and thixotropic properties of the liquid carrier. For example, the components may be selected such that the liquid carrier exhibits sufficient viscosity to suspend the particulate substance(s) over a storage period while maintaining suspension pourability. In some embodiments, the components are selected such that the liquid carrier exhibits sufficient viscosity to uniformly suspend the particulate substance(s), such that when the composition is in a container, there is <50%, <40%, <30%, <20%, or <10% variance in the particulate concentration of the composition at the top versus the bottom of the container.

In the present invention, the thixotropic agent is or includes a microcrystalline cellulose/sodium carboxymethylcellulose product such as Avicel^{®} RC 591. In the present invention, the thickening agent is xanthan gum.

The liquid carrier may further include other components besides thixotropic agents and thickening agents, such as antioxidants, chelating agents, preservatives, sweeteners, pH adjustment agents, colorants, flavoring agents, and water.

Non-limiting examples of antioxidants include glutathione, quinolines, polyphenols, carotenoids, sodium metabisulphite, tocopherol succinate, propyl gallate, butylated hydroxy toluene, butyl hydroxy anisole, flavonoids, and a vitamin C source. Non-limiting examples of vitamin C sources may include ascorbic acid; ascorbyl palmitate; dipalmitate L-ascorbate; sodium L-ascorbate-2-sulfate; an ascorbic salt, such as sodium, potassium, or calcium ascorbate; and mixtures thereof. In specific embodiments, the antioxidant is or includes sodium metabisulphite.

Non-limiting examples of chelating agents include ethylenediaminetetraacetic acid (EDTA) and citric acid, hydrates thereof, salts thereof, and hydrates of the salts thereof. Examples of such chelating agents include ethylenediaminetetraacetic acid disodium salt, ethylenediaminetetraacetic acid disodium salt dihydrate, and citric acid monohydrate. Various combinations of chelating agents can be used if desired. In specific embodiments, the chelating agent is or includes disodium EDTA.

Non-limiting examples of preservatives include C₁₂ to C₁₅ alkyl benzoates, alkyl p-hydroxybenzoates, aloe vera extract, ascorbic acid, benzalkonium chloride, benzoic acid, benzoic acid esters of C₉ to C₁₅ alcohols, butylated hydroxytoluene, castor oil, cetyl alcohols, chlorocresol, citric acid, cocoa butter, coconut oil, diazolidinyl urea, diisopropyl adipate, dimethyl polysiloxane, DMDM hydantoin, ethanol, fatty acids, fatty alcohols, hexadecyl alcohol, hydroxybenzoate esters, iodopropynyl butylcarbamate, isononyl iso-nonanoate, jojoba oil, lanolin oil, methylparaben, mineral oil, oleic acid, olive oil, polyethers, polyoxypropylene butyl ether, polyoxypropylene cetyl ether, potassium sorbate, silicone oils, sodium propionate, sodium benzoate, sodium bisulfite, disodium metabisulfite, sorbic acid, stearic fatty acid, vitamin E, vitamin E acetate and derivatives, esters, salts and mixtures thereof. In specific embodiments, the preservative is or includes sodium benzoate.

Non-limiting examples of sweeteners include rebaudioside A, rebaudioside B, rebaudioside C, rebaudioside D, rebaudioside E, rebaudioside F, dulcoside A, dulcoside B, rubusoside, stevia, stevioside, mogroside IV, mogroside V, Luo Han Guo sweetener, siamenoside, monatin and its salts (monatin SS, RR, RS, SR), curculin, glycyrrhizic acid and its salts, thaumatin, monellin, mabinlin, brazzein, hernandulcin, phyllodulcin, glycyphyllin, phloridzin, trilobatin, baiyunoside, osladin, polypodoside A, pterocaryoside A, pterocaryoside B, mukurozioside, phlomisoside I, periandrin I, abrsoside A, cyclocarioside I, sucralose, acesulfame potassium and other salts, aspartame, alitame, saccharin, neohesperidin dihydrochalcone, cyclamate, neotame, N-[N-[3-(3-hydroxy-4-methoxyphenyl)propyl]-L-α-aspartyl]-L-phenylalanine 1-methyl ester, N-[N-[3-(3-hydroxy-4-methoxyphenyl)-3-methylbutyl]-L-α-aspartyl]-L-phenylalanine 1-methyl ester, N-[N-[3-(3-methoxy-4-hydroxyphenyl)propyl]-L-α-aspartyl]-L-phenylalanine 1-methyl ester, and salts and combinations thereof. In specific embodiments, the sweeter is or includes sucralose.

Non-limiting examples of pH-adjusting agents include acidifying agents such as acetate buffers, benzoate buffers, borate buffers, citrate buffers, diisopropylamine buffers, hydrochloric acid, lactic acid buffers, perchloric acid, phosphate buffers, tartaric acid, propionate buffers, citric acid, and mixtures thereof. Typically, the pH-adjusting agent is useful for buffering at a pH of about 4-4.5. In specific embodiments, the pH-adjusting agent is or includes citric acid.

Non-limiting examples of colorants include commercially available pigments such as FD&C Blue #1 Aluminum Lake, FD&C Blue #2, other FD&C Blue colors, titanium dioxide, iron oxide, and/or combinations thereof. In specific embodiments, the colorant is or includes titanium dioxide.

Non-limiting examples of flavoring agents include berry flavor, root beer flavor, cream flavor, chocolate flavor, peppermint flavor, spearmint flavor, butterscotch flavor, and wintergreen flavor and combinations thereof. Suitable berry flavoring agents include black cherry, strawberry, cherry, blueberry, raspberry and the like. In specific embodiments, the flavoring agent is or includes butterscotch flavor.

In any embodiments, the liquid carrier may have a pH of from about 4 to about 4.5.

### Oral Liquid Pharmaceutical Compositions

As noted above, in accordance with the present invention, there are provided oral liquid pharmaceutical compositions comprising a plurality of extended release aminosalicylate microparticles provided with a delayed release coating (also referred to as "delayed/extended release aminosalicylate microparticles"), suspended in an aqueous liquid carrier formulated for oral administration. The delayed/extended release aminosalicylate microparticles can be any delayed/extended release aminosalicylate microparticles in accordance with the description above. The aqueous liquid carrier can be any aqueous liquid carrier in accordance with the description above.

In some embodiments, the pH of the oral liquid pharmaceutical composition is less than about 5, including less than 5, from about 4 to about 5, from about 4 to about 4.5, from 4 to 4.5, about 4, about 4.5, 4, or 5.

In some embodiments, the pH of the oral liquid pharmaceutical composition is selected and controlled so that any pH-dependent delayed release coating provided on the extended release aminosalicylate microparticles is substantially insoluble at that pH. For example, if the extended release aminosalicylate microparticles are provided with a pH-dependent delayed release coating that is substantially insoluble at a pH of about 5 or lower, the pH of the oral liquid pharmaceutical composition can be selected and controlled to be about 5 or lower, including less than 5, from about 4 to about 4.5, from 4 to 4.5, about 4, about 4.5, 4, or 5.

In specific embodiments, an oral liquid pharmaceutical composition is formulated to provide 2 g of aminosalicylate in a volume of about 30-60 mL, or in 60 mL or less, 55 mL or less, 50 mL or less, 45 mL or less, 40 mL or less, 35 mL or less, or 30 mL or less, such as in 60 mL, 55 mL, 50 mL, 45 mL, 40 mL, 35 mL, 30 mL, or 25 mL.

In specific embodiments, the oral liquid pharmaceutical composition is formulated to provide 2 g of mesalazine in a volume of about 30-60 mL, or in 60 mL or less, 55 mL or less, 50 mL or less, 45 mL or less, 40 mL or less, 35 mL or less, or 30 mL or less, such as in 60 mL, 55 mL, 50 mL, 45 mL, 40 mL, 35 mL, 30 mL, or 25 mL.

In one embodiment, the oral liquid pharmaceutical composition comprises about 4-8% w/w aminosalicylate, about 4-8% w/w poly(methacrylic acid-co-methyl methacrylate) 1:2, about 5-12% w/w cellulose acetate, about 0.8-1.2% w/w microcrystalline cellulose, about 0.05-0.1% w/w xanthan gum, and water.

In another embodiment, the oral liquid pharmaceutical composition comprises about 2-6% w/w aminosalicylate, about 6-10% w/w poly(methacrylic acid-co-methyl methacrylate) 1:2, about 5-12% w/w cellulose acetate butyrate, about 0.8-1.2% w/w microcrystalline cellulose, about 0.05-0.1% w/w xanthan gum, and water.

In another embodiment, the oral liquid pharmaceutical composition comprises about 4-5% w/w aminosalicylate, about 3.0-8.0% w/w poly(methacrylic acid-co-methyl methacrylate) 1:1, about 3-8% w/w poly(methacrylic acid-co-methyl methacrylate) 1:2, about 3-8% w/w cellulose acetate butyrate, about 0.5-1.5% w/w microcrystalline cellulose, about 0.05-0.1% w/w xanthan gum, and water.

In another embodiment, the oral liquid pharmaceutical composition comprises about 4-5% w/w aminosalicylate, about 4-15% w/w poly(methacrylic acid-co-methyl methacrylate) 1:1, about 1-5% w/w poly(methacrylic acid-co-methyl methacrylate) 1:2, about 3-9% w/w cellulose acetate butyrate, about 0.5-1.5% w/w microcrystalline cellulose, about 0.05-0.1% w/w xanthan gum, and water.

In some embodiments, the oral liquid pharmaceutical composition comprises about 4-5% w/w aminosalicylate, about 3% w/w poly(methacrylic acid-co-methyl methacrylate) 1:1, about 3% w/w poly(methacrylic acid-co-methyl methacrylate) 1:2, about 3.5% w/w cellulose acetate butyrate, about 1% w/w microcrystalline cellulose, about 0.1% w/w xanthan gum, and water.

In some embodiments, the oral liquid pharmaceutical composition comprises about 4-5% w/w aminosalicylate, about 4% w/w poly(methacrylic acid-co-methyl methacrylate) 1:1, about 4% w/w poly(methacrylic acid-co-methyl methacrylate) 1:2, about 4% w/w cellulose acetate butyrate, about 1% w/w microcrystalline cellulose, about 0.1% w/w xanthan gum, and water.

In some embodiments, the oral liquid pharmaceutical composition comprises about 4-5% w/w aminosalicylate, about 8% w/w poly(methacrylic acid-co-methyl methacrylate) 1:1, about 8% w/w poly(methacrylic acid-co-methyl methacrylate) 1:2, about 8% w/w cellulose acetate butyrate, about 1% w/w microcrystalline cellulose, about 0.05% w/w xanthan gum, and water.

In the present invention, the oral liquid pharmaceutical composition exhibits an *in vitro* dissolution profile such that, when subject to *in vitro* dissolution testing according to USP II Paddle at 37 °C and 100 rpm in 750 mL dissolution medium of pH 1.2 buffer for 2 hours, followed by pH 7 buffer for 12 hours, the composition exhibits substantially no release of aminosalicylate at pH 1.2, and release substantially all aminosalicylate within about 3 to about 8 hours, or within about 3 to about 7 hours, at pH 7. As used herein release of "substantially all" aminosalicylate refers to at least 75%, at least 80%, at least 85%, at least 90%, at least 95%, or 100% release, by weight.

In some embodiments, an oral liquid pharmaceutical composition as described herein is stable for at least 1 year or at least 2 years when stored at 25°C and 60% relative humidity.

In some embodiments, after storage for 2 years at 25 °C and 60% relative humidity, an oral liquid pharmaceutical composition as described herein exhibits an *in vitro* dissolution profile such that, when subject to *in vitro* dissolution testing according to USP II Paddle at 37 °C and 100 rpm in 750 mL dissolution medium of pH 1.2 buffer for 2 hours, followed by pH 7 buffer for 12 hours, the oral liquid pharmaceutical composition exhibits substantially no release of aminosalicylate at pH 1.2, and release substantially all aminosalicylate within about 3 to about 8 hours at pH 7. As used herein release of "substantially all" aminosalicylate refers to at least 75%, at least 80%, at least 85%, at least 90%, at least 95%, or 100% release, by weight.

In some embodiments, after storage for 2 years at 25 °C and 60% relative humidity, the amount of aminosalicylate degradants in the oral liquid pharmaceutical composition is less than 3% by weight of the original amount aminosalicylate in the composition. In some embodiments, after storage for 2 years at 25 °C and 60% relative humidity, the amount of aminosalicylate present in the delayed/extended release aminosalicylate microparticles of the oral liquid pharmaceutical composition differs by no more than 3% by weight from the original amount.

### Methods of Manufacture

Also provided herein are processes for making the extended release aminosalicylate microparticles, delayed/ extended release aminosalicylate microparticles, liquid carrier, and oral liquid pharmaceutical compositions as described herein.

In general, extended release aminosalicylate microparticles can be prepared by an emulsion solvent evaporation process. Emulsion solvent evaporation processes for making drug-containing particles are known in the art but have not been heretofore described for microparticles as described herein, or for microparticles containing a high proportion of an aminosalicylate as described herein.

In specific embodiments, the emulsion solvent evaporation process comprises dispersing an aminosalicylate in a solution comprising an extended release polymer to obtain a dispersed phase, emulsifying the dispersed phase in an immiscible solvent, and evaporating the solvent from the dispersed phase, to obtain a plurality of the extended release aminosalicylate microparticles comprising the aminosalicylate in a matrix comprising the extended release polymer.

In specific embodiments, the extended release polymer is any one or more of the extended release polymers described above and is prepared in a solution comprising any suitable solvent, such as acetonitrile and/or dichloromethane.

In specific embodiments, the immiscible solvent includes components typically used for the oil phase in an emulsion solvent evaporation, such as liquid paraffin and water and a surfactant. In other specific embodiments, the immiscible solvent is aqueous in character and includes polyvinyl alcohol (e.g., polyvinyl alcohol 4-88) and water.

Other details of an emulsion solvent evaporation process useful for making extended release aminosalicylate microparticles as described herein are illustrated in the examples below.

In some embodiments, the extended release aminosalicylate microparticles have a size such that, after being provided with a delayed release coating as discussed below, they have a Dx90 particle size of ≤ 250 µm, such as from about 100 µm to about 250 µm, including ≤ 250 µm, ≤ 200 µm, , ≤ 150 µm, and ≤ 100 µm. For example, in some embodiments, the extended release aminosalicylate microparticles have a Dx90 particle size of ≤ 150 µm, such as from about 50 µm to about 150 µm, including ≤ 150 µm, and ≤ 100 µm.

As noted above, the extended release aminosalicylate microparticles may be provided with a delayed release coating. The delayed release coating may be provided by any suitable process, including an emulsion solvent evaporation process or a fluid bed coating process.

In specific embodiments, the delayed release coating is provided using an emulsion solvent evaporation process that comprises dispersing the extended release aminosalicylate microparticles in a solution comprising delayed release polymer to obtain a dispersed phase, emulsifying the dispersed phase in an immiscible solvent, and evaporating the solvent from the dispersed phase, to obtain a plurality of extended release aminosalicylate microparticles with a delayed release coating.

In specific embodiments, the delayed release polymer is any one or more of the delayed release polymers described above and is prepared in a solution comprising any suitable solvent, such as ethanol.

In specific embodiments, the immiscible solvent includes components typically used for the oil phase in an emulsion solvent evaporation, such as liquid paraffin and a surfactant.

Other details of an emulsion solvent evaporation process useful for providing a delayed release coating on extended release aminosalicylate microparticles as described herein are illustrated in the examples below.

In other specific embodiments, the delayed release coating is provided using a fluid bed coating process. The fluid bed coating process can be controlled to provide a coating weight gain effective to achieve a target level of pH-dependent release. For example, a higher coating weight gain generally is associated with greater resistance to release at a pH below the pH at which the coating is soluble. For example, one or more delayed release polymers can be dissolved into a solution and spray-coated onto extended release aminosalicylate microparticles in a fluid bed coater. Additionally or alternatively, as discussed above, in some embodiments the delayed release coating is provided as more than one layers, each of which is applied sequentially, optionally drying a layer before a next layer is applied.

In specific embodiments, the delayed release coating is provided using a fluid bed coating process that comprises dissolving one or more delayed release polymers in a suitable solvent to obtain a delayed release polymer solution, and spray-coating the extended release aminosalicylate microparticles with the delayed release polymer solution in a fluid bed coater to obtain a plurality of extended release aminosalicylate microparticles with a delayed release coating. In some embodiments, the suitable solvent comprises acetone, ethanol, water, or a mixture thereof. In some embodiments, an anti-tacking agent and/or a plasticizer is added to the delayed release polymer solution. In some embodiments, the anti-tacking agent is talc. In some embodiments, the plasticizer is dibutyl sebacate. In some embodiments, a second delayed release coating comprising one or more delayed release polymers is applied to the delayed release polymer-coated microparticles, to provide a bilayer delayed release coating.

Optionally, delayed release polymer-coated microparticles can be cured, such as in an oven, to obtain delayed/extended release aminosalicylate microparticles, such as prior to being formulated in a liquid carrier. In some embodiments, the curing is effected in an oven. In some embodiments, the temperature for curing is from about 30 °C to about 150 °C, such as from about 50 °C to about 100 °C. In some embodiments, the curing time is from about 6 hours to about 48 hours, such as from about 16 hours to about 24 hours. In some embodiments, the curing is effected at a relative humidity of from about 50% RH to about 75% RH. In some embodiments, the coated microparticles are cured in an oven at 100°C for 16 hours. In some embodiments, the coated microparticles are cured in an oven at 50°C/75% RH for 24 hours. In some embodiments, curing improves the stability of the delayed/extended release aminosalicylate microparticles.

In some embodiments, the extended release aminosalicylate microparticles provided with a delayed release coating have a Dx90 particle size of ≤ 250 µm, as discussed above. That is, the delayed/extended release aminosalicylate microparticles have a Dx90 particle size of from about 100 µm to about 250 µm, or ≤ 250 µm, ≤ 200 µm, ≤ 150 µm, or ≤ 100 µm.

In general, an aqueous liquid carrier as described herein can be prepared by combining, mixing and/or blending the components in any suitable order. In specific embodiments, the thixotropic agent and thickening agent are added to water under stirring and homogenized, and then the other components are added under stirring. In other embodiments, the thixotropic agent is added to water under stirring and homogenized, and then the thickening agent is mixed in and homogenized. After homogenization, other components are added and homogenized. Suitable processes for making an aqueous liquid carrier as described herein is illustrated in the examples below.

In general, an oral liquid pharmaceutical composition as described herein can be made by suspending delayed/extended release aminosalicylate microparticles in a liquid carrier as described herein. In some embodiments, the process comprises (a) preparing a plurality of extended release aminosalicylate microparticles; (b) providing a delayed release coating on the extended release aminosalicylate microparticles to obtain the plurality of delayed/extended release aminosalicylate microparticles; and (c) suspending the plurality of delayed/extended release aminosalicylate microparticles in a liquid carrier. In some embodiments, the process further comprises prior to step (c), screening the delayed/extended release aminosalicylate microparticles to select delayed/extended release aminosalicylate microparticles having a Dx90 particle size of from about 100 µm to about 250 µm, or ≤ 250 µm, ≤ 200 µm, ≤ 150 µm, or ≤ 100 µm, and performing step (c) on the selected delayed/extended release aminosalicylate microparticles. In specific embodiments, step (b) further comprises curing the delayed/extended release aminosalicylate microparticles. In specific embodiments, step (c) comprises adding delayed/extended release aminosalicylate microparticles to the liquid carrier and mixing thoroughly.

Suitable processes for making an oral liquid pharmaceutical composition as described herein is illustrated in the examples below.

### Therapeutic Methods

Also provided herein are methods of administering an aminosalicylate to a subject in need thereof, comprising orally administering to the subject an oral liquid pharmaceutical composition as described herein. The subject may be suffering from or at risk of developing inflammatory bowel disease, including ulcerative colitis or Crohn's disease. The subject may be suffering from active inflammatory bowel disease or may be in remission. Thus, treating a subject includes reducing the symptoms and/or duration of active inflammatory bowel disease and increasing the length of remission periods (e.g., reducing the likelihood of flares).

Also provided herein are methods of treating inflammatory bowel disease, including ulcerative colitis or Crohn's disease, comprising orally administering to a subject in need thereof an oral liquid pharmaceutical composition as described herein. As noted above, a suitable subject may be suffering from one or more of ulcerative colitis, pancolitis, extensive colitis, backwash ileitis, and/or one or more of Crohn's disease, ileal disease, ileocolonic disease and colonic disease.

The methods may comprise administering the oral liquid pharmaceutical composition as described herein one or more times per day, such as one, two, three, four, five, or more, times per day.

Also provided are uses of an aminosalicylate in the preparation of a medicament for orally administering aminosalicylate to a subject in need thereof, wherein the medicament comprises an oral liquid pharmaceutical composition as described herein. Also provided are uses of aminosalicylate in the preparation of a medicament for treating inflammatory bowel disease, such as ulcerative colitis and mild-to-moderate Crohn's disease, wherein the medicament comprises an oral liquid pharmaceutical composition as described herein.

Also provided are oral liquid pharmaceutical compositions as described herein for orally administering aminosalicylate to a subject in need thereof. Also provided are oral liquid pharmaceutical compositions as described herein for treating inflammatory bowel disease, such as ulcerative colitis and mild-to-moderate Crohn's disease.

In some embodiments, the oral liquid pharmaceutical compositions described herein provide selective release of an aminosalicylate at a specific region of the digestive tract (e.g., the distal ileum or colon). For example, an oral liquid pharmaceutical compositions as described herein may, after oral administration, release most of its active agent in the distal ileum and/or colon, such as by not releasing more than 25 % w/w, more than 30 % w/w, more than about 40 % w/w, or more than about 50 % w/w of the total amount of active agent until the composition reaches that specific region of the digestive tract, and/or by releasing more than about 50 % w/w, more than about 60 % w/w, more than about 70 % w/w or more than about 75 % w/w of the total amount of active agent in the composition in that region of the digestive tract.

### EXAMPLES

The following specific examples are included as illustrative of the compositions described herein. These examples are in no way intended to limit the scope of the disclosure. Other aspects of the disclosure will be apparent to those skilled in the art to which the disclosure pertains.

The following procedures can be used to produce compositions and unit dose pharmaceutical products described above.

### Example 1A: Extended-Release Aminosalicylate Microparticles

Extended release aminosalicylate microparticles described herein can be made by an oil-in-oil emulsion solvent evaporation process. An exemplary emulsion solvent evaporation process is set forth below.

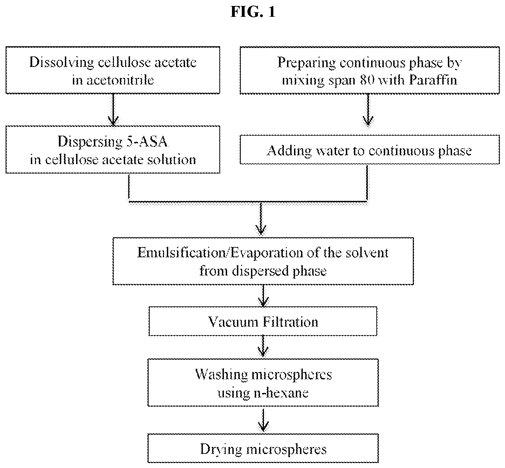

Cellulose acetate was added to acetonitrile and stirred (700 rpm, magnetic stirrer) until completely dissolved. Mesalazine was added to the cellulose acetate solution and stirred (1000 rpm, magnetic stirrer) to obtain a uniform suspension. A mixture of liquid paraffin containing surfactant (sorbitan oleate, Span^{®}-80) was prepared and cooled to -2 °C at 800 rpm for 2 hours. The mesalazine suspension was poured into the liquid paraffin under constant stirring (1600 rpm, variable speed propeller) at -2 °C for a period of 20 hours, then stirred (1000 rpm, variable speed propeller) at room temperature for 7 hours to remove acetonitrile, thereby obtaining solidified microparticles comprising mesalazine in a cellulose acetate matrix. The solidified microparticles were vacuum filtered, washed, and dried in an oven at 50 °C for 20 hours, to obtain dry extended release mesalazine microparticles. These steps are illustrated in FIG. 1. The amounts of components used are set forth in the table below.

| Material | Formula (g) |
|---|---|
| Cellulose acetate | 20.00 |
| Mesalazine | 13.35 |
| Acetonitrile | 286.22 |
| Span^{®} 80 | 16.20 |
| Paraffin | 2003.60 |
| Water | 40.75 |
| Total | 2380.00 |

The particle size of four batches of extended release mesalazine microparticles prepared as described above was assessed by laser diffraction. Results are reported below.

### Particle Size Distribution of ER Microparticles

| Batch # | Dx10 µm | Dx50 µm | Dx90 µm |
|---|---|---|---|
| 1 | 64.8 | 80.2 | 98.4 |
| 2 | 63.3 | 77.4 | 94.3 |
| 3 | 60.6 | 73.8 | 90.4 |
| 4 | 61.8 | 75.3 | 92.0 |

A similar process can be used to make other extended release aminosalicylate microparticles using different extended release polymers and/or different aminosalicylates.

### Example 1B: Extended-Release Aminosalicylate Microparticles

Extended release aminosalicylate microparticles described herein can be made by an oil-in-water emulsion solvent evaporation process. An exemplary emulsion solvent evaporation process is set forth below.

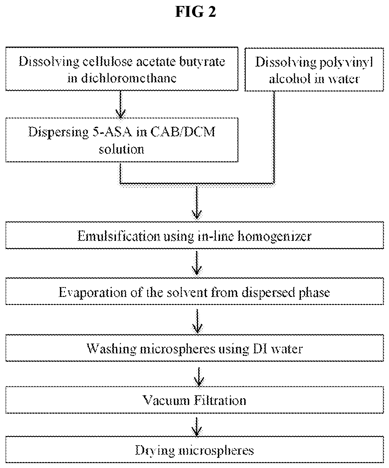

Cellulose acetate butyrate was added to dichloromethane and stirred (800 rpm, magnetic stirrer) until completely dissolved. Mesalazine was added to the cellulose acetate butyrate solution and stirred (800 rpm, magnetic stirrer) to obtain a uniform suspension. An aqueous solution containing emulsifier (polyvinyl alcohol 4-88) was prepared. The mesalazine suspension was poured into the polyvinyl alcohol 4-88 solution in a Chemglass reactor under constant stirring (200 rpm, variable speed propeller), while circulating (1kg/min fluid flow rate) through a homogenizer (950 rpm) at room temperature for a period of 15 minutes. The emulsion was then transferred into deionized water pre-cooled to 10°C and stirred (200 rpm, variable speed propeller) at 10°C for 20 hours to remove dichloromethane, thereby obtaining solidified microparticles comprising mesalazine in a cellulose acetate butyrate matrix. The solidified microparticles were washed, vacuum filtered, and dried in an oven at 50 °C for 20 hours, to obtain dry extended release mesalazine microparticles. These steps are illustrated in FIG. 2. The amounts of components used are set forth in the table below.

| Ingredient | Formula of Example 1B |
|---|---|
| Cellulose acetate butyrate (g) | 16 |
| Mesalamine (g) | 16 |
| Dichloromethane in DP (mL) | 160 |
| Polyvinyl alcohol 4-88 (g) | 8 |
| DI water (g) | 800 |

The particle size of four batches of extended release mesalazine microparticles prepared as described above was assessed by laser diffraction. Results are reported below.

### Particle Size Distribution of ER Microparticles of Example 1B

| Example | Dx10 (um) | Dx50 (um) | Dx90 (um) |
|---|---|---|---|
| Example 1A | 58.0 | 90.0 | 135 |

A similar process can be used to make other extended release aminosalicylate microparticles using different extended release polymers and/or different aminosalicylates.

### Example 2A: Delayed/Extended-Release Aminosalicylate Microparticles

Delayed/extended release aminosalicylate microparticles described herein can be made by providing a delayed release coating on extended release aminosalicylate microparticles using an emulsion solvent evaporation process. An exemplary emulsion solvent evaporation process is set forth below.

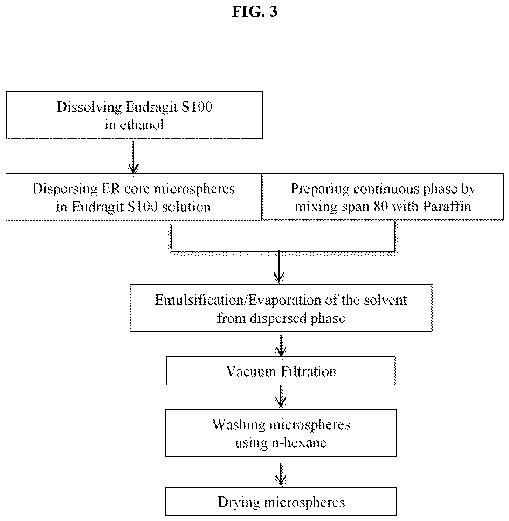

Extended release aminosalicylate (mesalazine) microparticles prepared as described in Example 1A were provided with a delayed release coating comprised of Eudragit^{®} S-100 using an emulsion solvent evaporation process as described in Example 1A. Specifically, Eudragit^{®} S 100 was dissolved in ethanol (700 rpm, magnetic stirrer). Extended release mesalazine microparticles were added to the Eudragit^{®} S100 solution (1000 rpm, magnetic stirrer) to obtain a uniform suspension. A mixture of liquid paraffin containing surfactant (sorbitan oleate, Span^{®}-80) was prepared and cooled to 10 °C at 1000 rpm for 2 hours. The microparticle suspension was poured into the liquid paraffin under maintained stirring (1400 rpm, variable speed propeller) at 10 °C for a period of 20 hours, then stirred (1000 rpm, variable speed propeller) at room temperature for 5 hours to completely remove ethanol, thereby obtaining solidified coated microparticles. The solidified microparticles were vacuum filtered, washed, and dried in an oven at 50 °C for 20 hours. These steps are illustrated in FIG. 3. The relative amounts and batch amounts of components used are set forth in the table below.

| Material | Composition (%) | Formula (g) |
|---|---|---|
| Eudragit^{®} S100 | 0.874 | 1.00 |
| ER Microparticles | 2.19 | 2.50 |
| Ethanol (200 proof) | 6.90 | 7.88 |
| Span^{®} 80 | 2.62 | 3.00 |
| Paraffin | 87.4 | 100.0 |
| Total | 100 | |

The particle size of a batch of delayed/extended release mesalazine microparticles prepared as described above was determined to be: D×10 = 94.6 µm; Dx50 = 151 µm; Dx90 = 233 µm.

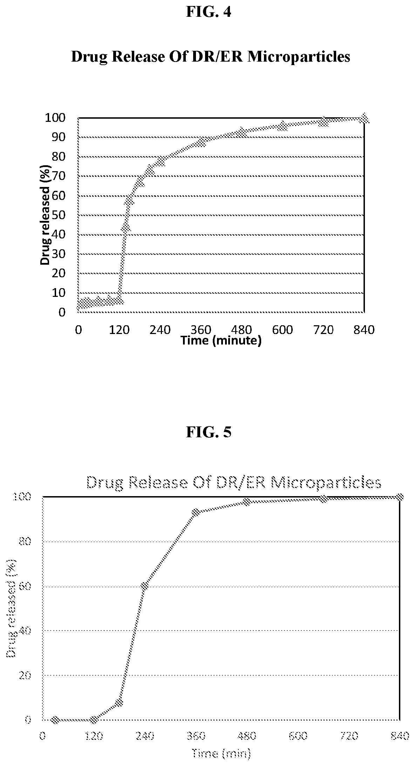

The *in vitro* dissolution profile of delayed/extended release mesalazine microparticles prepared as described above was assessed by two-stage *in vitro* dissolution testing according to USP II Paddle at 37 °C and 100 rpm in 750 mL dissolution medium of pH 1.2 buffer for 2 hours, followed by pH 7 buffer for 12 hours. Results are set forth in FIG. 4.

A similar process can be used to make other delayed/extended release aminosalicylate microparticles using different delayed release polymers and/or different aminosalicylate microparticles.

### Example 2B: Delayed/Extended-Release Aminosalicylate Microparticles

Delayed/extended release aminosalicylate microparticles described herein can be made by providing a delayed release coating on extended release aminosalicylate microparticles using a fluid bed coating process. An exemplary fluid bed coating process is set forth below.

Extended release aminosalicylate (mesalazine) microparticles prepared as described in Example 1B were provided with a delayed release coating comprised of Eudragit^{®} S-100 using a fluid bed coating process. Specifically, Eudragit S-100^{®} was slowly added into a mixture of acetone, ethanol and water while stirring. Then, talc and dibutyl sebacate were added and the mixture was stirred for 10 minutes with an overhead mixer to make a delayed release polymer suspension.

The delayed release polymer suspension was sprayed on microparticles loaded in a Mini-Glatt fluid bed coater and coated at a product temperature of 25°C. Then, the coated microparticles were cured in an oven at 100°C for 16 hours. The composition of final DR/ER microparticles (which has a coating weight gain of 100%) are set forth in the table below.

| Material | Composition (%) |
|---|---|
| Eudragit^{®} S100 | 50 |
| ER Microparticles | 50 |
| Total | 100 |

The *in vitro* dissolution profile of delayed/extended release mesalazine microparticles prepared as described above was assessed by two-stage *in vitro* dissolution testing according to USP II Paddle at 37 °C and 100 rpm in 750 mL dissolution medium of pH 1.2 buffer for 2 hours, followed by pH 7 buffer for 12 hours. Results are set forth in FIG. 5.

A similar process can be used to make other delayed/extended release aminosalicylate microparticles using different delayed release polymers and/or different aminosalicylate microparticles.

### Example 2C: Delayed/Extended-Release Aminosalicylate Microparticles

Delayed/extended release aminosalicylate microparticles described herein can be made using two delayed release polymers. Exemplary fluid bed coating processes are set forth below.

Extended release aminosalicylate (mesalazine) microparticles as described herein were provided with a delayed release single layer coating comprising Eudragit^{®} L-100 and Eudragit^{®} S-100 in a 1:1 ratio using a fluid bed coating process. Specifically, Eudragit^{®} L-100 and Eudragit^{®} S-100 were slowly added into a mixture of acetone, ethanol and water while stirring. Stirring for 10 minutes with an overhead mixer provided a delayed release polymer solution.

The composition of the coating solution used to prepare DR microparticles with a single layer coating of Eudragit^{®} L-100 and Eudragit^{®} S-100 (1:1) is provided below:

| Material | Composition (%) |
|---|---|
| Eudragit^{®} L100 | 2.25 |
| Eudragit^{®} S100 | 2.25 |
| DI water | 6.50 |
| Acetone | 36.65 |
| Ethanol | 52.35 |
| Total | 100 |

Extended release aminosalicylate (mesalazine) microparticles as described herein were provided with a delayed release bilayer coating comprising Eudragit^{®} L-100 and Eudragit^{®} S-100 at an 80:20 ratio using a fluid bed coating process. Each coating (Eudragit^{®} L-100 or Eudragit^{®} S-100) was prepared as described above.

The compositions of the coating solutions used to prepare the bilayer coating are provided below:

| Material | Eudragit^{®} L-100 80% weight gain Composition (%) | Eudragit^{®} S-100 20% weight gain Composition (%) |
|---|---|---|
| Eudragit^{®} L100 | 4.5 | N/A |
| Eudragit^{®} S100 | N/A | 4.5 |
| DI water | 6.50 | 6.50 |
| Acetone | 36.65 | 36.65 |
| Ethanol | 52.35 | 52.35 |
| Total | 100 | 100 |

The delayed release polymer suspensions as described above were sprayed onto extended release microparticles loaded in a Mini-Glatt fluid bed coater and coated at a product temperature of 25°C. To prepare the bilayer coating, the Eudragit^{®} L-100 polymer suspension was applied first, followed by drying, following by application of the Eudragit^{®} S-100 polymer solution. For some samples, the coated microparticles were cured in an oven at 50°C/75% RH for 24 hours prior to formulating in the liquid carrier.

A similar process can be used to make other delayed/extended release aminosalicylate microparticles using different delayed release polymers and/or different aminosalicylate microparticles.

### Example 3A: Aqueous Liquid Carrier

An exemplary process for making an aqueous liquid carrier as described herein is set forth below.

The thixotropic agent and thickening agent (Avicel^{®} RC591 and xanthan gum) were added to water under propeller mixing at 1200 rpm for 5 minutes. The mixture was then homogenized using a homogenizer at 3500 rpm for 10 minutes. Then, other components (sodium metabisulfite, disodium edetate, sodium benzoate, sucralose, citric acid (anhydrous) and butterscotch flavor) were added under mixing with propeller mixer at 1200 rpm for 20 minutes. Then titanium dioxide was added under mixing at 800 rpm for 10 minutes. The relative amounts and batch amounts of components used are set forth in the table below.

### Liquid Carrier

| Material | (%) | (g) |
|---|---|---|
| Avicel^{®} RC591 | 1.24 | 15.0 |
| Xanthan Gum | 0.01 | 1.20 |
| Sodium metabisulfite | 0.02 | 0.24 |
| Disodium edetate (EDTA) | 0.02 | 0.24 |
| Sodium benzoate | 0.30 | 3.60 |
| Sucralose | 0.10 | 1.20 |
| Citric acid, anhydrous | 0.19 | 2.29 |
| Titanium dioxide | 0.40 | 4.81 |
| Butterscotch flavor | 0.20 | 2.40 |
| Water | 97.44 | 1177 |
| Total | 100.0 | 1209 |

A similar process can be used to make other aqueous liquid carriers using different components and/or amounts thereof.

### Example 3B: Aqueous Liquid Carrier

Another exemplary process for making an aqueous liquid carrier as described herein is set forth below.

The thixotropic agent (Avicel^{®} RC591) was added to a vessel containing deionized water under propeller mixing at 750 rpm for 10 minutes. The mixture was then homogenized using a high shear mixer with a duplex mixing assembly at 4000 rpm for 15 minutes. The thickening agent (Xanthan gum) was added under homogenization at 4000 rpm for 10 minutes, followed by mixing at 750 rpm for 10 minutes under propeller mixing to assure complete dispersion of thickening agent. The other components (sodium metabisulfite, disodium edetate, sodium benzoate, sucralose, and citric acid (anhydrous)) were added under homogenization at 4000 rpm for 15 minutes. The titanium dioxide was screened and weighed into the vessel under homogenization at 4000 rpm for 10 minutes. The pH of the suspension was adjusted to 4.0. The relative amounts and batch amounts of components used are set forth in the table below.

### Liquid Carrier

| Material | (%) |
|---|---|
| Avicel^{®} RC591 | 1.25 |
| Xanthan gum 180 | 0.10 |
| Sodium benzoate | 0.30 |
| Sodium metabisulphite | 0.15 |
| Disodium EDTA | 0.02 |
| Sucralose | 0.10 |
| Citric acid anhydrous | 0.19 |
| Titanium dioxide | 0.40 |
| Water | 97.49 |
| Total | 100.0 |

A similar process can be used to make other aqueous liquid carriers using different components and/or different amounts thereof.

### Example 4: Oral Liquid Pharmaceutical Composition (Invention Example)

An exemplary process for making oral liquid pharmaceutical compositions as described herein is set forth below.

Delayed/extended release aminosalicylate (mesalazine) microparticles made as described above in Example 2A were added to liquid carrier made as described above in Example 3 and mixed to obtain a homogeneous suspension, thereby obtaining an oral liquid pharmaceutical composition.

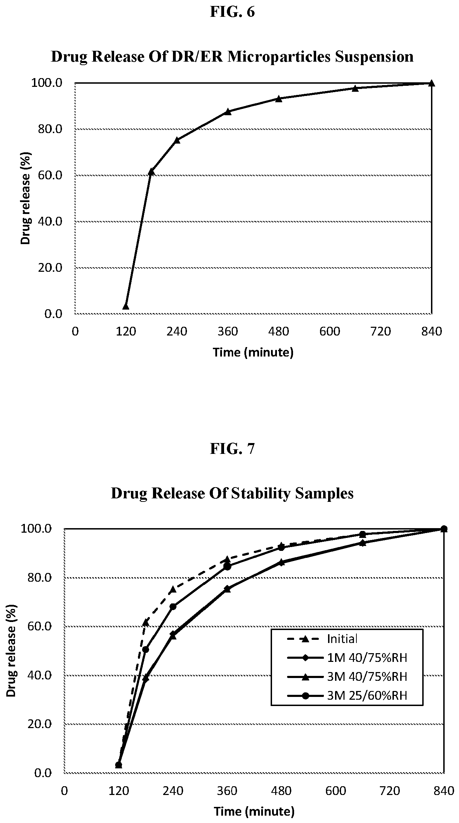

The *in vitro* dissolution profile of the 500 mg oral liquid pharmaceutical compositions prepared as described above was assessed by two-stage *in vitro* dissolution testing according to USP II Paddle at 37 °C and 100 rpm in 750 mL dissolution medium of pH 1.2 buffer for 2 hours, followed by pH 7 buffer for 12 hours. Results are set forth in FIG. 6.

### Example 5: Stability of Oral Liquid Pharmaceutical Composition

The stability of the oral liquid pharmaceutical composition made as described in Example 4 was assessed after storage at 40°C/75% Relative Humidity (RH) for 1 and 3 months or 25°C/60%RH for 3 months. At each sampling time point, 100 mg samples were tested for drug content and degradation. Results are set forth below.

| | | 40°C/75%RH | | 25°C/60%RH |
|---|---|---|---|---|
| | Initial | 1M | 3M | 3M |
| Assay | 91.30 | 92.30 | 92.20 | 94.40 |
| RRT = 0.33 | NA | NA | 0.24 | NA |
| RRT = 1.67 | NA | 0.09 | 0.28 | NA |
| Total deg | < Quantitation limit | 0.09 | 0.52 | < Quantitation limit |

The accelerated stability studies (at 40°C/75%RH) showed the formation of degradation products but were less than 1% after 3 months.

The dissolution profile was assessed by two-stage *in vitro* dissolution testing according to USP II Paddle at 37 °C and 100 rpm in 750 mL dissolution medium of pH 1.2 buffer for 2 hours, followed by pH 7 buffer for 12 hours. Results are set forth in FIG. 7. The results indicate that dissolution rate slowed after 1 month of storage under accelerated conditions, with a 10% difference observed at 240 minutes and 360 minutes. However, the dissolution rate after storage under accelerated conditions for 3 months was not changed as compared to storage after 1 month. Also, the dissolution rate did not change after the samples were stored at 25°C/60%RH for three months.

Leakage of mesalazine into the liquid carrier also was assessed. Results are reported in the table below.

| T=0 | 1M | 3M | 3M |
|---|---|---|---|
| | 40/75%RH | 40/75%RH | 25/60%RH |
| 0.21% | 0.27% | 0.21% | 0.29% |

At all time points, less than 0.30% of mesalazine was leaked into the liquid carrier from the microparticles.

### Example 6: Dissolution Studies

Additional *in vitro* dissolution studies were assessed as follows:
ER Microparticles (without DR coating): USP Apparatus II (Paddle) at 37 °C and 100 rpm in 500 mL dissolution medium of pH 7.2 KH₂PO₄ + NaOH buffer. A continuous loop UV system was used to analyze samples. A 45µm PE filter was used.

DR Microparticles suspend in liquid vehicle just prior to testing (two stage): USP Apparatus II (Paddle) at 37 °C and 100 rpm in 750 mL dissolution medium of (1) pH 1.2 0.1 N HCl for 2 hours, followed by (ii) addition of 250 mL of preheated 0.4 M KH₂PO₄ + 0.5 M NaOH to provide a dissolution medium of 1000 mL of pH 7.2 for 12 hours. Samples collected at all time points were then analyzed by HPLC.

### 50% Mesalazine Extended Release Core

50% extended release mesalazine microparticles (having a core comprised of 50% w/w mesalazine) were prepared by an oil-in-water emulsion solvent evaporation process generally as described in Example 1B having the following composition:

| | |
|---|---|
| Ingredient | 50% Core |
| Cellulose acetate butyrate (g) | 16 |
| Mesalamine (g) | 16 |
| Dichloromethane in DP (mL) | 160 |
| Polyvinyl alcohol 4-88 (g) | 8 |
| DI water (g) | 800 |

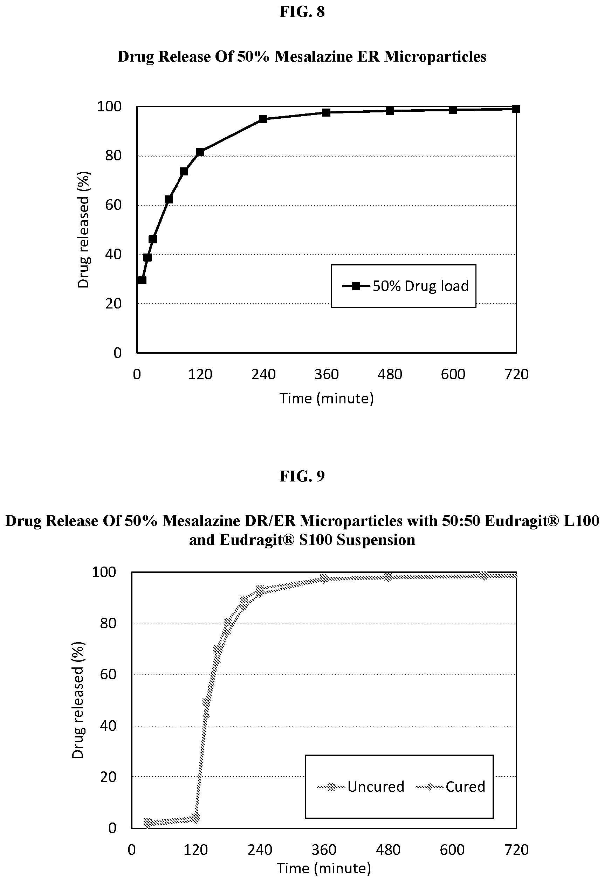

The dissolution profile is set forth in FIG. 8.

50% extended release mesalazine microparticles were coated with a delayed release single layer coating containing a 1:1 mixture of Eudragit^{®} L-100 and Eudragit^{®} S-100 applied at a 100% weight gain by a fluid bed coating process generally as described in Example 2C. The resulting delayed/extended release microparticles were either cured or not cured as described in Example 2C before being suspended in an aqueous liquid carrier as described in Example 3B. The two stage dissolution profiles are set forth in FIG. 9.

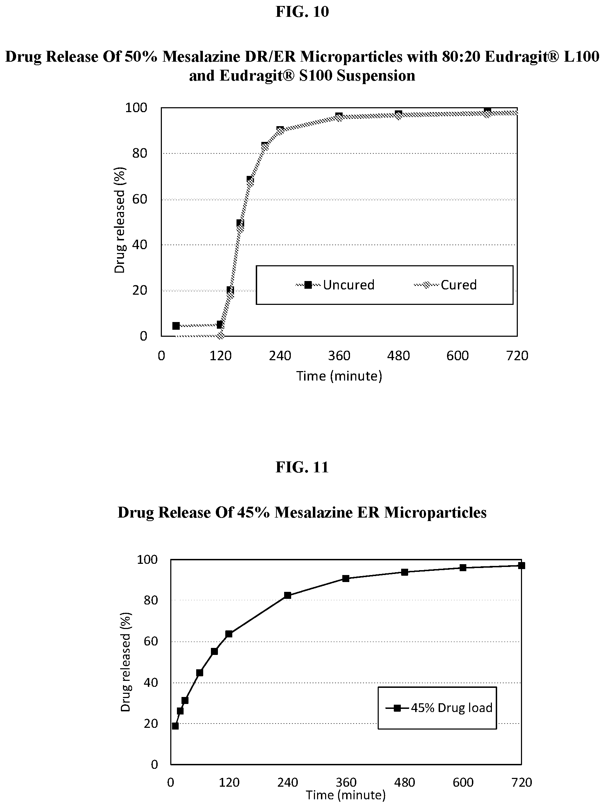

In another example, 50% extended release mesalazine microparticles were coated with a delayed release bilayer coating containing Eudragit^{®} L-100 (applied at an 80% weight gain relative to the ER microparticles) and Eudragit^{®} S-100 (applied at a 20% weight gain relative to the ER microparticles) by a fluid bed coating process generally as described in Example 2C. The resulting delayed/extended release microparticles were either cured or not cured as described in Example 2C before being suspended in an aqueous liquid carrier as described in Example 3B. The two-stage dissolution profiles are set forth in FIG. 10.

### 45% Mesalazine Extended Release Core

Similar studies were conducted with a core comprising 45% w/w mesalazine, prepared and coated generally as described above, having the following composition:

| | |
|---|---|
| Ingredient | 45% Core |
| Cellulose acetate butyrate (g) | 16 |
| Mesalamine (g) | 13.1 |
| Dichloromethane in DP (mL) | 160 |
| Polyvinyl alcohol 4-88 (g) | 8 |
| DI water (g) | 800 |

The dissolution profile of the 45% ER microparticles (without the delayed release coating) is set forth in FIG. 11.

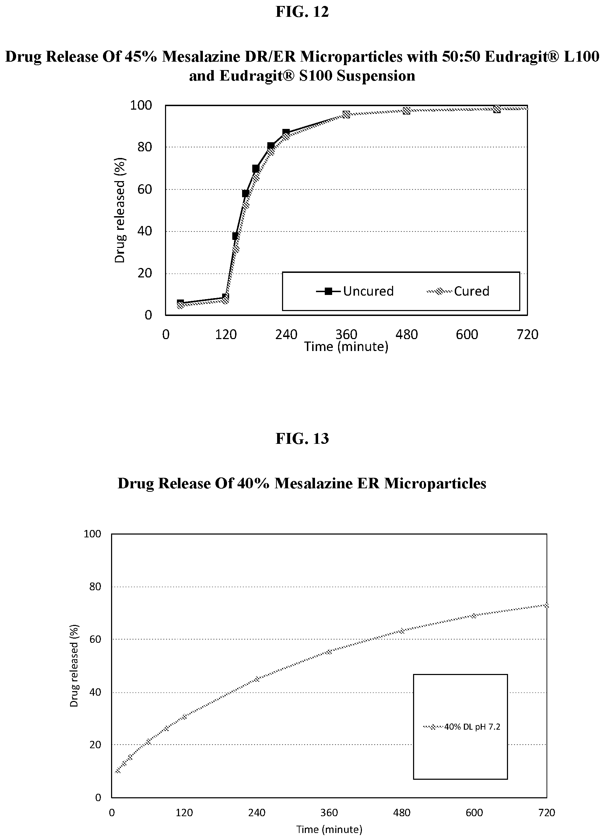

The two-stage dissolution profiles of 45% ER microparticles coated with a delayed release single layer coating containing a 1:1 mixture of Eudragit^{®} L-100 and Eudragit^{®} S-100 (applied at a 100% weight gain) and cured or not cured before being suspended in an aqueous liquid carrier are set forth in FIG. 12.

The two-stage dissolution profiles of 45% ER microparticles coated with a delayed release bilayer coating containing Eudragit^{®} L-100 (applied at an 80% weight gain relative to the ER microparticles) and Eudragit^{®} S-100 (applied at a 20% weight gain relative to the ER microparticles) and cured or not cured before being suspended in an aqueous liquid carrier also were determined (data not shown).

### 40% Mesalazine Extended Release Core

Similar studies were conducted with a core comprising 40% w/w mesalazine, prepared and coated generally as described above, having the following composition:

| | |
|---|---|
| Ingredient | 40% Core |
| Cellulose acetate butyrate (g) | 64 |
| Mesalamine (g) | 42.7 |
| Dichloromethane in DP (mL) | 640 |
| Polyvinyl alcohol 4-88 (g) | 32 |
| DI water (g) | 3200 |

The dissolution profile of the 40% ER microparticles (without the delayed release coating) is set forth in FIG. 13.

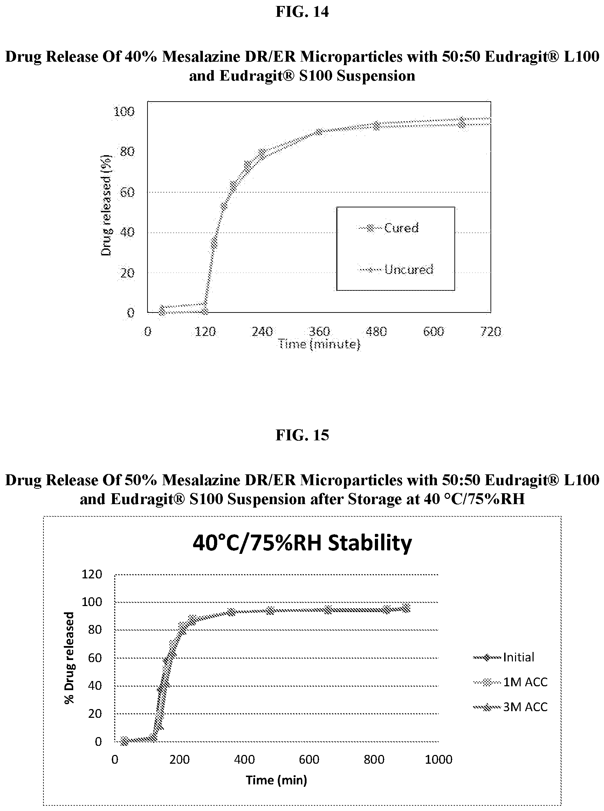

The two-stage dissolution profiles of 40% ER microparticles coated with a delayed release single layer coating containing a 1:1 mixture of Eudragit^{®} L-100 and Eudragit^{®} S-100 (applied at a 100% weight gain) and cured or not cured before being suspended in an aqueous liquid carrier are set forth in FIG. 14.

### Example 7: Stability Studies

The stability of the liquid compositions made as described in Example 6 was assessed after storage at 40°C/75% Relative Humidity (RH) for 1 and 3 months, 30°C/65% Relative Humidity (RH) for 3 months, or 25°C/60%RH for 3 months. At each sampling time point, 100 mg samples were tested for drug content and degradation and dissolution profiles of initial, 1 month and 3 month samples were compared.

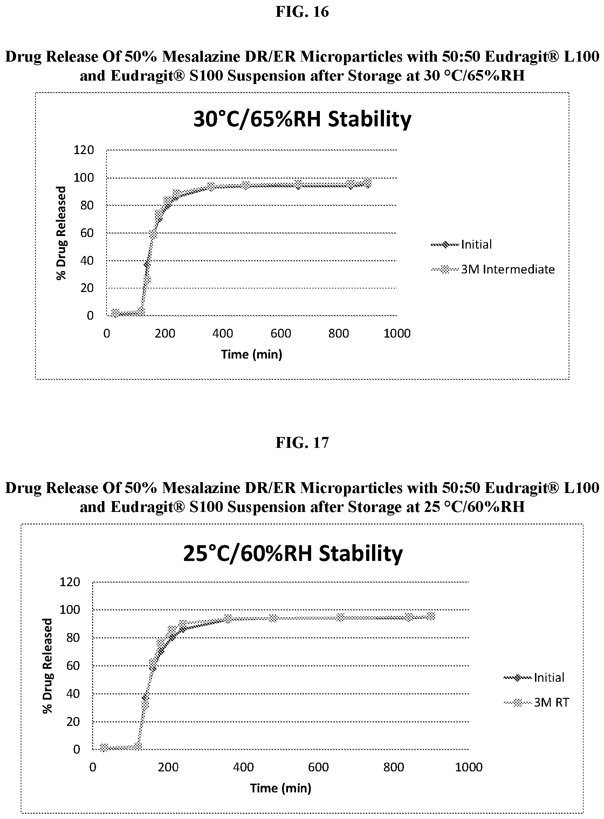

The microparticles provided with a single layer coating and cured prior to formulation in the liquid carrier exhibited good stability. *See* FIGs. 15-17. Microparticles provided with a bilayer coating exhibited poorer stability. Microparticles not cured prior to formulation in the liquid carrier exhibited slightly poorer stability than cured counterparts.

### Example 8: Illustrative Formulations

The following table sets forth illustrative formulations, including components of the liquid carrier. The "50% DL core" had a core comprised of 50% w/w 5-ASA, with the delayed release coating applied at a weight gain of 100% w/w. The "35% DL core" had a core comprised of 35% w/w 5-ASA, with the delayed release coating applied at a weight gain of 125% w/w. The "55% DL core" had a core comprised of 55% w/w 5-ASA, with the delayed release coating applied at a weight gain of 75% w/w.

| **Ingredient** | **50% ER Core** | **35% ER Core** | **55% ER Core** |
|---|---|---|---|
| | **100% Weight Gain** | **125% Weight Gain** | **75% Weight Gain** |
| **Liquid Carrier** | | | |
| Avicel^{®} RC591 | 1.03 | 0.89 | 1.07 |
| Xanthan gum 180 | 0.08 | 0.07 | 0.09 |
| Sodium benzoate | 0.25 | 0.21 | 0.26 |
| Sodium metabisulphite | 0.12 | 0.11 | 0.13 |
| Disodium EDTA | 0.02 | 0.01 | 0.02 |
| Sucralose | 0.08 | 0.07 | 0.09 |
| Citric acid (anhydrous) | 0.16 | 0.14 | 0.16 |
| Titanium dioxide | 0.33 | 0.29 | 0.34 |
| Water | 80.16 | 69.65 | 83.69 |

| **Delayed Release (DR) Coating** | | | |
|---|---|---|---|
| Eudragit^{®} L100 | 4.44 | 7.93 | 3.03 |
| Eudragit^{®} S100 | 4.44 | 7.93 | 3.03 |

| **Extended Release (ER) Core** | | | |
|---|---|---|---|
| Mesalamine | 4.44 | 4.44 | 4.45 |
| Cellulose Acetate Butyrate | 4.44 | 8.25 | 3.64 |
| **Total** | 100.00 | 100.00 | 100.00 |

## Claims

1. An oral liquid pharmaceutical composition comprising a plurality of extended release aminosalicylate microparticles provided with an enteric delayed release coating ("delayed/extended release aminosalicylate microparticles"), suspended in an aqueous liquid carrier formulated for oral administration, wherein the extended release aminosalicylate microparticles comprise an aminosalicylate formulated in an extended release matrix comprising an extended release polymer comprising one or more selected from cellulose acetate polymers and cellulose acetate butyrate polymers,
wherein the enteric delayed release coating comprises one or more delayed release polymers selected from copolymers of methacrylic acid and methyl methacrylate,
wherein the aqueous liquid carrier comprises a thixotropic agent selected from microcrystalline cellulose and a thickening agent selected from xanthan gum,
wherein the oral liquid composition exhibits an *in vitro* dissolution profile such that, when subject to *in vitro* dissolution testing according to USP II Paddle at 37 °C and 100 rpm in 750 mL dissolution medium of pH 1.2 buffer for 2 hours, followed by pH 7 buffer for 12 hours, the oral liquid pharmaceutical composition exhibits substantially no release of aminosalicylate at pH 1.2, and release substantially all aminosalicylate within 3 to 8 hours ±10% at pH 7; and
wherein the oral liquid composition is selected from an oral liquid pharmaceutical composition that comprises:
(a) 4-8% w/w ± 10% aminosalicylate, 4-8% w/w ±10% poly(methacrylic acid-co-methyl methacrylate) 1:2, 5-12% w/w ±10% cellulose acetate, 0.8-1.2% w/w ±10% microcrystalline cellulose, 0.05-0.1% w/w ±10% xanthan gum, and water;
(b) 2-6% w/w ± 10% aminosalicylate, 6-10% w/w ±10% poly(methacrylic acid-co-methyl methacrylate) 1:2, 5-12% w/w ±10% cellulose acetate butyrate, 0.8-1.2% w/w ±10% microcrystalline cellulose, 0.05-0.1% w/w ±10% xanthan gum, and water;
(c) 4-5% w/w ±10% aminosalicylate, 3-8% w/w ±10% poly(methacrylic acid-co-methyl methacrylate) 1:1, 3-8% w/w ±10% poly(methacrylic acid-co-methyl methacrylate) 1:2, 3-8% w/w ±10% cellulose acetate butyrate, 0.5-1.5% w/w ± 10% microcrystalline cellulose, 0.05-0.1% w/w ±10% xanthan gum, and water; or
(d) 4-5% w/w ± 10% aminosalicylate, 4-15% w/w ±10% poly(methacrylic acid-co-methyl methacrylate) 1:1, 1-5% w/w ± 10% poly(methacrylic acid-co-methyl methacrylate) 1:2, 3-9% w/w ±10% cellulose acetate butyrate, 0.5-1.5% w/w ±10% microcrystalline cellulose, 0.05-0.1% w/w ±10% xanthan gum, and water.

2. The oral liquid pharmaceutical composition of claim 1, wherein the Dx90 particle size of the delayed/extended release microparticles is selected from ≤ 250 µm, ≤ 200 µm, ≤ 150 µm, and ≤ 100 µm.

3. The oral liquid pharmaceutical composition of claim 1 or 2, wherein the aminosalicylate comprises one or more selected from mesalazine, 4-aminosalicylic acid, balsalazide, olsalazine, sulfasalazine, and pharmaceutically acceptable salts of each thereof.

4. The oral liquid pharmaceutical composition of any of claims 1-3, comprising 4-8% w/w ±10% aminosalicylate, 4-8% w/w ±10% poly(methacrylic acid-co-methyl methacrylate) 1:2, 5-12% w/w ±10% cellulose acetate, 0.8-1.2% w/w ±10% microcrystalline cellulose, 0.05-0.1% w/w ±10% xanthan gum, and water.

5. The oral liquid pharmaceutical composition of any of claims 1-3, comprising 2-6% w/w ±10% aminosalicylate, 6-10% w/w ±10% poly(methacrylic acid-co-methyl methacrylate) 1:2, 5-12% w/w ±10% cellulose acetate butyrate, 0.8-1.2% w/w ±10% microcrystalline cellulose, 0.05-0.1% w/w ±10% xanthan gum, and water.

6. The oral liquid pharmaceutical composition of any of claims 1-3, comprising 4-5% w/w ±10% aminosalicylate, 3-8% w/w ±10% poly(methacrylic acid-co-methyl methacrylate) 1:1, 3-8% w/w ±10% poly(methacrylic acid-co-methyl methacrylate) 1:2, 3-8% w/w ±10% cellulose acetate butyrate, 0.5-1.5% w/w ±10% microcrystalline cellulose, 0.05-0.1% w/w ±10% xanthan gum, and water.

7. The oral liquid pharmaceutical composition of any of claims 1-3, comprising 4-5% w/w ±10% aminosalicylate, 4-15% w/w ±10% poly(methacrylic acid-co-methyl methacrylate) 1:1, 1-5% w/w ±10% poly(methacrylic acid-co-methyl methacrylate) 1:2, 3-9% w/w ±10% cellulose acetate butyrate, 0.5-1.5% w/w ±10% microcrystalline cellulose, 0.05-0.1% w/w ±10% xanthan gum, and water.

8. The oral liquid pharmaceutical composition of any one of the preceding claims, wherein the composition exhibits a stability such that:
(a) the composition is stable for at least 1 year or at least 2 years when stored at 25°C and 60% relative humidity; and/or
(b) after storage for 2 years at 25 °C and 60% relative humidity, the oral liquid pharmaceutical composition exhibits an *in vitro* dissolution profile such that, when subject to *in vitro* dissolution testing according to USP II Paddle at 37 °C and 100 rpm in 750 mL dissolution medium of pH 1.2 buffer for 2 hours, followed by pH 7 buffer for 12 hours, the oral liquid pharmaceutical composition exhibits substantially no release of aminosalicylate at pH 1.2, and release substantially all aminosalicylate within 3 to 8 hours ±10% at pH 7; and/or
(c) after storage for 2 years at 25 °C and 60% relative humidity, the amount of aminosalicylate degradants in the oral liquid pharmaceutical composition is less than 3% by weight of the original amount aminosalicylate in the oral liquid pharmaceutical composition; and/or
(d) after storage for 2 years at 25 °C and 60% relative humidity, the amount of aminosalicylate present in the microparticles of the oral liquid pharmaceutical composition differs by no more than 3% by weight from the original amount.

9. A process for making an oral liquid pharmaceutical aminosalicylate composition as defined in any one of claims 1 to 8 and comprising a plurality of delayed/extended release aminosalicylate microparticles suspended in an aqueous liquid carrier, comprising:
(a) preparing a plurality of extended release aminosalicylate microparticles comprising an aminosalicylate formulated in an extended release matrix comprising an extended release polymer comprising one or more selected from cellulose acetate polymers and cellulose acetate butyrate polymers;
(b) providing a delayed release coating comprising one or more delayed release polymers selected from copolymers of methacrylic acid and methyl methacrylate on the extended release aminosalicylate microparticles to obtain the plurality of delayed/extended release aminosalicylate microparticles; and
(c) suspending the plurality of delayed/extended release aminosalicylate microparticles in an aqueous liquid carrier comprising a thixotropic agent and a thickening agent, and
wherein the oral liquid composition exhibits an *in vitro* dissolution profile such that, when subject to *in vitro* dissolution testing according to USP II Paddle at 37 °C and 100 rpm in 750 mL dissolution medium of pH 1.2 buffer for 2 hours, followed by pH 7 buffer for 12 hours, the oral liquid pharmaceutical composition exhibits substantially no release of aminosalicylate at pH 1.2, and release substantially all aminosalicylate within 3 to 8 hours ±10% at pH 7.

10. An oral liquid pharmaceutical composition according to any one of claims 1-8, which is adapted for oral administration.

11. An oral liquid pharmaceutical composition according to any one of claims 1-8 for use in treating inflammatory bowel disease, such as ulcerative colitis and mild-to-moderate Crohn's disease.

## Patentansprüche

1. Orale flüssige pharmazeutische Zusammensetzung, umfassend eine Vielzahl von Aminosalicylat-Mikropartikeln mit verlängerter Freisetzung, die mit einer enterisch-verzögerte-Freisetzung-Beschichtung (enteric delayed release coating) versehen sind ("Aminosalicylat-Mikropartikel mit verzögerter/verlängerter Freisetzung" - "delayed/extended release aminosalicylate microparticles"), suspendiert in einem wässrigen flüssigen Träger, der für die orale Verabreichung formuliert ist, wobei die Aminosalicylat-Mikropartikel mit verlängerter Freisetzung umfassen ein Aminosalicylat, formuliert in einer Matrix mit verlängerter Freisetzung, umfassend ein Polymer mit verlängerter Freisetzung, umfassend ein oder mehrere, ausgewählt aus Celluloseacetat-Polymeren und Celluloseacetat-Butyrat-Polymeren,
wobei die enterisch-verzögerte-Freisetzung-Beschichtung umfasst ein oder mehrere Polymere mit verzögerter Freisetzung, ausgewählt aus Copolymeren von Methacrylsäure und Methylmethacrylat,
wobei der wässrige flüssige Träger umfasst ein thixotropes Mittel, ausgewählt aus mikrokristalliner Cellulose und ein Verdickungsmittel ausgewählt aus Xanthangummi,
wobei die orale flüssige Zusammensetzung ein solches in-vitro-Auflösungsprofil (*in vitro* dissolution profile) aufweist, dass, wenn sie einem in-vitro-Auflösungstest gemäß USP II Paddle bei 37 °C und 100 U/min in 750 ml Auflösungsmedium aus pH 1,2-Puffer für 2 Stunden, gefolgt von pH 7-Puffer für 12 Stunden, unterzogen wird, die orale flüssige pharmazeutische Zusammensetzung im Wesentlichen keine Freisetzung von Aminosalicylat bei pH 1,2 aufweist und im Wesentlichen das gesamte Aminosalicylat innerhalb von 3 bis 8 Stunden ±10% bei pH 7 freisetzt; und
wobei die orale flüssige Zusammensetzung ausgewählt ist aus einer oralen flüssigen pharmazeutischen Zusammensetzung, die umfasst:
(a) 4-8% Gew./Gew. 110% Aminosalicylat, 4-8% Gew./Gew. 110% Poly(methacrylsäure-co-methylmethacrylat) 1:2, 5-12% Gew./Gew. 110% Celluloseacetat, 0,8-1,2% Gew./Gew. 110% mikrokristalline Cellulose, 0,05-0,1% Gew./Gew. 110% Xanthangummi, und Wasser;
(b) 2-6% Gew./Gew. 110% Aminosalicylat, 6-10% Gew./Gew. 110% Poly(methacrylsäure-co-methylmethacrylat) 1:2, 5-12% Gew./Gew. 110% Celluloseacetatbutyrat, 0,8-1,2% Gew./Gew. 110% mikrokristalline Cellulose, 0,05-0,1% Gew./Gew. 110% Xanthangummi und Wasser;
(c) 4-5% Gew./Gew. 110% Aminosalicylat, 3-8% Gew./Gew. 110% Poly(methacrylsäure-co-methylmethacrylat) 1:1, 3-8% Gew./Gew. 110% Poly(methacrylsäure-co-methylmethacrylat) 1:2, 3-8% Gew./Gew. 110% Celluloseacetatbutyrat, 0,5-1,5% Gew./Gew. ±10% mikrokristalline Cellulose, 0,05-0,1% Gew./Gew. 110% Xanthangummi, und Wasser; oder
(d) 4-5% Gew./Gew. 110% Aminosalicylat, 4-15% Gew./Gew. 110% Poly(methacrylsäure-co-methylmethacrylat) 1:1, 1-5% Gew./Gew. 110% Poly(methacrylsäure-co-methylmethacrylat) 1:2, 3-9% Gew./Gew. 110% Celluloseacetatbutyrat, 0,5-1,5% Gew./Gew. ±10% mikrokristalline Cellulose, 0,05-0,1% Gew./Gew. 110% Xanthangummi, und Wasser.

2. Orale flüssige pharmazeutische Zusammensetzung gemäß Anspruch 1, wobei die Dx90-Partikelgröße der Mikropartikel mit verzögerter/verlängerter Freisetzung ausgewählt ist aus ≤ 250 µm, ≤ 200 µm, ≤ 150 µm und ≤ 100 µm.

3. Orale flüssige pharmazeutische Zusammensetzung gemäß Anspruch 1 oder 2, wobei das Aminosalicylat umfasst eines oder mehrere, ausgewählt aus Mesalazin, 4-Aminosalicylsäure, Balsalazid, Olsalazin, Sulfasalazin, sowie deren jeweilige pharmazeutisch akzeptablen Salze.

4. Orale flüssige pharmazeutische Zusammensetzung gemäß mindestens einem der Ansprüche 1-3, umfassend 4-8% Gew./Gew. ± 10 % Aminosalicylat, 4-8% Gew./Gew. ± 10% Poly(methacrylsäure-co-methylmethacrylat) 1:2, 5-12% Gew./Gew. ± 10% Celluloseacetat, 0,8-1,2% Gew./Gew. ± 10% mikrokristalline Cellulose, 0,05-0,1% Gew./Gew. ± 10% Xanthangummi, und Wasser.

5. Orale flüssige pharmazeutische Zusammensetzung gemäß mindestens einem der Ansprüche 1-3, umfassend 2-6% Gew./Gew. ± 10% Aminosalicylat, 6-10% Gew./Gew. ± 10% Poly(methacrylsäure-co-methylmethacrylat) 1:2, 5-12% Gew./Gew. ± 10% Celluloseacetatbutyrat, 0,8-1,2% Gew./Gew. ± 10% mikrokristalline Cellulose, 0,05-0,1% Gew./Gew. ± 10% Xanthangummi, und Wasser.

6. Orale flüssige pharmazeutische Zusammensetzung gemäß mindestens einem der Ansprüche 1-3, umfassend 4-5% Gew./Gew. ± 10% Aminosalicylat, 3-8% Gew./Gew. ± 10% Poly(methacrylsäure-co-methylmethacrylat) 1:1, 3-8% Gew./Gew. ± 10% Poly(methacrylsäure-co-methylmethacrylat) 1:2, 3-8% Gew./Gew. ± 10% Celluloseacetatbutyrat, 0,5-1,5% Gew./Gew. 110% mikrokristalline Cellulose, 0,05-0,1% Gew./Gew. 110% Xanthangummi, und Wasser.

7. Orale flüssige pharmazeutische Zusammensetzung gemäß mindestens einem der Ansprüche 1-3, umfassend 4-5% Gew./Gew. ± 10% Aminosalicylat, 4-15% Gew./Gew. ± 10% Poly(methacrylsäure-co-methylmethacrylat) 1:1, 1-5% Gew./Gew. ± 10% Poly(methacrylsäure-co-methylmethacrylat) 1:2, 3-9% Gew./Gew. 110% Celluloseacetatbutyrat, 0,5-1,5% Gew./Gew. 110% mikrokristalline Cellulose, 0,05-0,1% Gew./Gew. 110% Xanthangummi und Wasser.

8. Orale flüssige pharmazeutische Zusammensetzung gemäß mindestens einem der vorhergehenden Ansprüche, wobei die Zusammensetzung eine solche Stabilität aufweist, dass:
(a) die Zusammensetzung für mindestens 1 Jahr oder mindestens 2 Jahre stabil ist, wenn sie bei 25°C und 60% relativer Luftfeuchtigkeit gelagert wird; und/oder
(b) die orale flüssige pharmazeutische Zusammensetzung nach 2 Jahren Lagerung bei 25 °C und 60 % relativer Luftfeuchtigkeit ein solches in-vitro-Auflösungsprofil aufweist, dass die orale flüssige pharmazeutische Zusammensetzung, wenn sie einem in-vitro-Auflösungstest gemäß USP II Paddle bei 37 °C und 100 U/min in 750 ml Auflösungsmedium aus pH 1,2-Puffer für 2 Stunden, gefolgt von pH 7-Puffer für 12 Stunden, unterzogen wird, im Wesentlichen keine Freisetzung von Aminosalicylat bei pH 1,2 zeigt und im Wesentlichen das gesamte Aminosalicylat innerhalb von 3 bis 8 Stunden ±10 % bei pH 7 freisetzt; und/oder
(c) nach 2 Jahren Lagerung bei 25 °C und 60 % relativer Luftfeuchtigkeit die Menge der Aminosalicylat-Abbaustoffe in der oralen flüssigen pharmazeutischen Zusammensetzung weniger als 3 Gew.-% der ursprünglichen Aminosalicylatmenge in der oralen flüssigen pharmazeutischen Zusammensetzung beträgt; und/oder
(d) nach 2 Jahren Lagerung bei 25 °C und 60 % relativer Luftfeuchtigkeit die Menge an Aminosalicylat, die in den Mikropartikeln der oralen flüssigen pharmazeutischen Zusammensetzung vorhanden ist, um nicht mehr als 3 Gew.-% von der ursprünglichen Menge abweicht.

9. Verfahren zur Herstellung einer oralen flüssigen pharmazeutischen Aminosalicylat-Zusammensetzung, wie sie in mindestens einem der Ansprüche 1 bis 8 definiert ist und umfassend eine Vielzahl von Aminosalicylat-Mikropartikeln mit verzögerter/verlängerter Freisetzung, suspendiert in einem wässrigen flüssigen Träger, umfassend:
(a) Herstellen einer Vielzahl von Aminosalicylat-Mikropartikeln mit verlängerter Freisetzung, umfassend ein Aminosalicylat, das in einer Matrix mit verlängerter Freisetzung formuliert ist umfassend ein Polymer mit verlängerter Freisetzung umfassend ein oder mehrere ausgewählt aus Celluloseacetat-Polymeren und Celluloseacetat-Butyrat-Polymeren;
(b) Bereitstellen einer Beschichtung mit verzögerter Freisetzung umfassend ein oder mehrere Polymere mit verzögerter Freisetzung, ausgewählt aus Copolymeren von Methacrylsäure und Methylmethacrylat, auf den Aminosalicylat-Mikropartikeln mit verlängerter Freisetzung, um die Vielzahl von Aminosalicylat-Mikropartikeln mit verzögerter/verlängerter Freisetzung zu erhalten; und
(c) Suspendieren der Vielzahl von Aminosalicylat-Mikropartikeln mit verzögerter/verlängerter Freisetzung in einem wässrigen flüssigen Träger umfassend ein thixotropes Mittel und ein Verdickungsmittel, und
wobei die orale flüssige Zusammensetzung ein solches in-vitro-Auflösungsprofil aufweist, dass die orale flüssige pharmazeutische Zusammensetzung, wenn sie einem in vitro-Auflösungstest gemäß USP II Paddle bei 37 °C und 100 U/min in 750 ml Auflösungsmedium aus pH 1,2-Puffer für 2 Stunden, gefolgt von pH 7-Puffer für 12 Stunden, unterzogen wird, im Wesentlichen keine Freisetzung von Aminosalicylat bei pH 1,2 zeigt und im Wesentlichen das gesamte Aminosalicylat innerhalb von 3 bis 8 Stunden ±10% bei pH 7 freisetzt.

10. Orale flüssige pharmazeutische Zusammensetzung gemäß mindestens einem der Ansprüche 1-8, die zur oralen Verabreichung geeignet ist.

11. Orale flüssige pharmazeutische Zusammensetzung gemäß mindestens einem der Ansprüche 1-8 zur Verwendung bei der Behandlung von entzündlichen Darmerkrankungen, wie Colitis ulcerosa und leichtem bis mittelschwerem Morbus Crohn.

## Revendications

1. Composition pharmaceutique liquide orale comprenant une pluralité de microparticules d'aminosalicylate à libération prolongée dotées d'un enrobage entérique à libération différée ("microparticules d'aminosalicylate à libération différée/prolongée"), suspendues dans un vecteur liquide aqueux formulé pour administration orale, dans laquelle les microparticules d'aminosalicylate à libération prolongée comprennent un aminosalicylate formulé dans une matrice à libération prolongée comprenant un polymère à libération prolongée comprenant un ou plusieurs polymères sélectionnés parmi des polymères d'acétate de cellulose et des polymères de butyrate d'acétate de cellulose,
dans laquelle l'enrobage entérique à libération différée comprend un ou plusieurs polymères à libération différée sélectionnés parmi des copolymères d'acide méthacrylique et de méthacrylate de méthyle,
dans laquelle le vecteur liquide aqueux comprend un agent thixotropique sélectionné parmi de la cellulose microcristalline et un agent épaississant sélectionné parmi la gomme de xanthane,
dans laquelle la composition liquide orale présente un profil de dissolution *in vitro* de telle sorte que, lorsqu'elle est soumise à un test de dissolution *in vitro* selon USP II Paddle à 37 °C et 100 tr/min dans 750 ml de milieu de dissolution de tampon à pH 1,2 pendant 2 heures, suivi d'un tampon de pH 7 pendant 12 heures, la composition pharmaceutique liquide orale ne présente pratiquement aucune libération d'aminosalicylate au pH 1,2, et une libération de pratiquement la totalité d'aminosalicylate dans les 3 à 8 heures ±10 % au pH 7 ; et
dans laquelle la composition liquide orale est sélectionnée parmi une composition pharmaceutique liquide orale qui comprend :
(a) 4-8 % p/p ±10 % d'aminosalicylate, 4-8 % p/p ±10 % de poly(méthacrylique acide-co-méthacrylate de méthyle) 1:2, 5-12 % p/p ±10 % d'acétate de cellulose, 0,8-1,2 % p/p ±10 % de cellulose microcristalline, 0,05-0,1 % p/p ±10 % de gomme de xanthane, et de l'eau ;
(b) 2-6 % p/p ±10 % d'aminosalicylate, 6-10 % p/p ±10 % de poly(méthacrylique acide-co-méthacrylate de méthyle) 1:2, 5-12 % p/p ±10 % de butyrate d'acétate de cellulose, 0,8-1,2 % p/p ±10 % de cellulose microcristalline, 0,05-0,1 % p/p ±10 % de gomme de xanthane, et de l'eau ;
(c) 4-5 % p/p ±10 % d'aminosalicylate, 3-8 % p/p ±10 % de poly(méthacrylique acide-co-méthacrylate de méthyle) 1:1, 3-8 % p/p ±10 % de poly(méthacrylique acide-co-méthacrylate de méthyle) 1:2, 3-8 % p/p ±10 % de butyrate d'acétate de cellulose, 0,5-1,5 % p/p ±10 % de cellulose microcristalline, 0,05-0,1 % p/p ±10 % de gomme de xanthane, et de l'eau ; ou
(d) 4-5 % p/p ±10 % d'aminosalicylate, 4-15 % p/p ±10 % de poly(méthacrylique acide-co-méthacrylate de méthyle) 1:1, 1-5 % p/p ±10 % de poly(méthacrylique acide-co-méthacrylate de méthyle) 1:2, 3-9 % p/p ±10 % de butyrate d'acétate de cellulose, 0,5-1,5 % p/p ±10 % de cellulose microcristalline, 0,05-0,1 % p/p ±10 % de gomme de xanthane, et de l'eau.

2. Composition pharmaceutique liquide orale selon la revendication 1, dans laquelle la taille de particule Dx90 des microparticules à libération différée/prolongée est sélectionnée parmi ≤ 250 µm, ≤ 200 µm , ≤ 150 µm et ≤ 100 µm.

3. Composition pharmaceutique liquide orale selon la revendication 1 ou la revendication 2, dans laquelle l'aminosalicylate comprend un ou plusieurs sélectionnés composés parmi la mésalazine, l'acide 4-aminosalicylique, la balsalazide, l'olsalazine, la sulfasalazine et des sels pharmaceutiquement acceptables de chacun de ceux-ci.

4. Composition pharmaceutique liquide orale selon l'une quelconque des revendications 1 à 3, comprenant 4-8 % p/p ±10 % d'aminosalicylate, 4-8 % p/p ±10 % de poly(méthacrylique acide-co-méthacrylate de méthyle) 1:2, 5-12 % p/p ±10 % d'acétate de cellulose, 0,8-1,2 % p/p ±10 % de cellulose microcristalline, 0,05-0,1 % p/p ±10 % de gomme de xanthane, et de l'eau.

5. Composition pharmaceutique liquide orale selon l'une quelconque des revendications 1 à 3, comprenant 2-6 % p/p ±10 % d'aminosalicylate, 6-10 % p/p ±10 % de poly(méthacrylique acide-co-méthacrylate de méthyle) 1:2, 5-12 % p/p ±10 % de butyrate d'acétate de cellulose, 0,8-1,2 % p/p ±10 % de cellulose microcristalline, 0,05-0,1 % p/p ±10 % de gomme de xanthane, et de l'eau.

6. Composition pharmaceutique liquide orale selon l'une quelconque des revendications 1 à 3, comprenant 4-5 % p/p ±10 % d'aminosalicylate, 3-8 % p/p ±10 % de poly(méthacrylique acide-co-méthacrylate de méthyle) 1:1, 3-8 % p/p ±10 % de poly(méthacrylique acide-co-méthacrylate de méthyle) 1:2, 3-8 % p/p ±10 % de butyrate d'acétate de cellulose, 0,5-1,5 % p/p ±10 % de cellulose microcristalline, 0,05-0,1 % p/p ±10 % de gomme de xanthane, et de l'eau.

7. Composition pharmaceutique liquide orale selon l'une quelconque des revendications 1 à 3, comprenant 4-5 % p/p ±10 % d'aminosalicylate, 4-15 % p/p ±10 % de poly(méthacrylique acide-co-méthacrylate de méthyle) 1:1, 1-5 % p/p ±10 % de poly(méthacrylique acide-co-méthacrylate de méthyle) 1:2, 3-9 % p/p ±10 % de butyrate d'acétate de cellulose, 0,5-1,5 % p/p ±10 % de cellulose microcristalline, 0,05-0,1 % p/p ±10 % de gomme de xanthane, et de l'eau.

8. Composition pharmaceutique liquide orale selon l'une quelconque des revendications précédentes, dans laquelle la composition présente une stabilité telle que :
(a) la composition est stable pendant au moins 1 an ou au moins 2 ans lorsqu'elle est stockée à 25 °C et 60 % d'humidité relative ; et/ou
(b) après stockage pendant 2 ans à 25 °C et 60 % d'humidité relative, la composition pharmaceutique liquide orale présente un profil de dissolution *in vitro* de telle sorte que, lorsqu'elle est soumise à un test de dissolution *in vitro* selon USP Il Paddle à 37 °C et 100 tr/min dans 750 ml de milieu de dissolution de tampon à pH 1,2 pendant 2 heures, suivi d'un tampon de pH 7 pendant 12 heures,
la composition pharmaceutique liquide orale ne présente pratiquement aucune libération d'aminosalicylate au pH 1,2, et une libération de pratiquement la totalité d'aminosalicylate dans les 3 à 8 heures ±10 % au pH 7 ; et/ou
(c) après stockage pendant 2 ans à 25 °C et 60 % d'humidité relative, la quantité de dégradants d'aminosalicylate dans la composition pharmaceutique liquide orale est inférieure à 3 % en poids de la quantité d'origine d'aminosalicylate dans la composition pharmaceutique liquide orale ; et/ou
(d) après stockage pendant 2 ans à 25 °C et 60 % d'humidité relative, la quantité d'aminosalicylate présente dans les microparticules de la composition pharmaceutique liquide orale diffère de pas plus de 3 % en poids de la quantité d'origine.

9. Processus de fabrication d'une composition d'aminosalicylate pharmaceutique liquide orale telle que définie dans l'une quelconque des revendications 1 à 8 et comprenant une pluralité de microparticules d'aminosalicylate à libération différée/prolongée suspendues dans un vecteur liquide aqueux, comprenant :
(a) la préparation d'une pluralité de microparticules d'aminosalicylate à libération prolongée comprenant un aminosalicylate formulé dans une matrice à libération prolongée comprenant un polymère à libération prolongée comprenant un ou plusieurs polymères sélectionnés parmi des polymères d'acétate de cellulose et des polymères de butyrate d'acétate de cellulose ;
(b) la fourniture d'un enrobage à libération différée comprenant un ou plusieurs polymères à libération différée sélectionnés parmi des copolymères d'acide méthacrylique et de méthacrylate de méthyle sur les microparticules d'aminosalicylate à libération prolongée pour obtenir la pluralité de microparticules d'aminosalicylate à libération différée/prolongée ; et
(c) la mise en suspension de la pluralité de microparticules d'aminosalicylate à libération différée/prolongée dans un vecteur liquide aqueux comprenant un agent thixotropique et un agent épaississant, et
dans lequel la composition liquide orale présente un profil de dissolution *in vitro* de telle sorte que, lorsqu'elle est soumise à un test de dissolution *in vitro* selon USP Il Paddle à 37 °C et 100 tr/min dans 750 ml de milieu de dissolution de tampon à pH 1,2 pendant 2 heures, suivi d'un tampon de pH 7 pendant 12 heures, la composition pharmaceutique liquide orale ne présente pratiquement aucune libération d'aminosalicylate au pH 1,2, et une libération de pratiquement la totalité d'aminosalicylate dans les 3 à 8 heures ±10 % au pH 7.

10. Composition pharmaceutique liquide orale selon l'une quelconque des revendications 1 à 8, qui est adaptée pour administration orale.

11. Composition pharmaceutique liquide orale selon l'une quelconque des revendications 1 à 8 pour utilisation dans le traitement d'une maladie inflammatoire de l'intestin, telle que la colite ulcéreuse et la maladie de Crohn légère à modérée.
